# FASCICULE DE BREVET EUROPEEN

(11) **EP 3 487 474 B1**
(45) Date de publication et mention de la délivrance du brevet: **08.09.2021**
(21) Numéro de dépôt: 17754402.0
(22) Date de dépôt: 21.07.2017
(51) Int. Cl.: A61K 8/49, A61Q 17/04, A61Q 19/08

(54) **PROCÉDÉ DE TRAITEMENT COSMÉTIQUE DE LA PEAU**
KOSMETISCHES BEHANDLUNGSVERFAHREN FÜR DIE HAUT
COSMETIC TREATMENT METHOD FOR THE SKIN

(30) Priorité: 22.07.2016 FR 1657014
(43) Date de publication de la demande: 29.05.2019
(73) Titulaire: Syntivia, 31100 Toulouse (FR)
(72) Inventeur: BEDOS, Philippe, 31450 Donneville (FR)
(74) Mandataire: Ipside
(86) Numéro de dépôt international: PCT/FR2017/052007
(87) Numéro de publication internationale: WO 2018/015688

(56) Documents cités:
- WO-A2-2006/005455
- WO-A2-2013/087834
- DE-U1- 20 110 355
- JP-A- 2008 239 545
- Anonymous: "Vitamin B6 - Healthy Skin Depends on this Vitamin", , 10 juillet 2011 (2011-07-10), XP055328321, Extrait de l'Internet: URL:http://vitamedica.com/wellness-blog/vi tamin-b-6/ [extrait le 2016-12-12]

## Description

La présente invention s'inscrit dans le domaine des compositions cosmétiques, notamment à usage topique, destinées à la protection et/ou au traitement de la peau, notamment pour prévenir et/ou réparer les effets du vieillissement cutané, en particulier liés aux agressions externes. Plus particulièrement, la présente invention concerne un procédé de traitement cosmétique de la peau mettant en œuvre un composé répondant à une formule générale particulière, ainsi que l'utilisation cosmétique d'un tel composé, et une composition cosmétique contenant un tel composé.

La peau est la première barrière protégeant le corps contre les agressions externes. Le phénomène de vieillissement cutané, qu'il soit naturel ou induit par ces agressions externes, affaiblit cette fonction barrière. En particulier, l'exposition aux agressions externes fragilise la peau et induit plusieurs processus de dégénérescence qui accélèrent le vieillissement cutané, notamment : des réactions inflammatoires, l'activation de la production de radicaux libres, néfastes pour le métabolisme cutané, la stimulation d'enzymes attaquant la peau en profondeur, telles que les métalloprotéases matricielles (MMP, pour l'anglais Matrix Metalloprotease), les dommages à l'ADN induits par le rayonnement ultraviolet, qui entrainent une instabilité génomique et peuvent conduire à la mort prématurée des cellules, etc.

Parmi ces agressions externes, on peut notamment citer les agressions par le rayonnement ultraviolet, l'exposition aux polluants, tels que les métaux lourds, la fumée de cigarette, etc.

Les symptômes du vieillissement cutané sont notamment l'apparition de rides, la sécheresse, la rugosité, l'amincissement et la perte d'élasticité de la peau. Le traitement de ces symptômes est devenu un enjeu majeur pour l'industrie cosmétique.

La connaissance de la physiologie de la peau a permis de proposer des solutions cosmétiques à différents dysfonctionnements induits par les agressions externes. Il a ainsi été proposé par l'art antérieur diverses compositions cosmétiques, contenant des principes actifs de types variés, pour prévenir et réparer les effets du vieillissement de la peau.

Parmi les principes actifs cosmétiques proposés par l'art antérieur pour améliorer l'aspect de la peau, on peut citer par exemple les composés d'origine végétale tels que les polyphénols, dont l'activité antioxydante a été mise à profit pour prévenir le stress oxydatif au niveau de la peau, par piégeage des radicaux libres résultant d'agressions extérieures, par exemple d'une exposition de la peau aux rayons ultraviolets (UV). L'acide caféique est un exemple d'un tel polyphénol mis en œuvre dans des compositions cosmétiques dites anti-âge proposées par l'art antérieur, par exemple illustré par le document JP 2008/239545. Les compositions cosmétiques à base de tels composés antioxydants permettent, dans une certaine mesure, de ralentir l'apparition des symptômes du vieillissement. Leur efficacité est cependant limitée, notamment par rapport à certains types d'agressions externes.

La présente invention vise à proposer un procédé de traitement cosmétique de la peau, mettant en œuvre une composition à usage topique, qui permette de protéger et/ou réparer efficacement la peau par rapport aux effets du vieillissement cutané liés aux agressions externes.

Il a maintenant été découvert par les présents inventeurs qu'une certaine classe de composés, répondant à une formule générale particulière, permettent, mis en œuvre en tant que principes actifs dans des compositions cosmétiques, en particulier à usage topique, d'atteindre un tel résultat, et notamment de protéger efficacement la peau contre les agressions externes.

Ainsi, selon un premier aspect, la présente invention concerne un procédé de traitement cosmétique, non-thérapeutique, de la peau d'un individu, en particulier pour prévenir et/ou réparer les effets du vieillissement de la peau, notamment dus aux agressions externes. Ce procédé comprend l'administration audit individu d'une composition contenant, dans un véhicule cosmétiquement acceptable, un composé de formule générale (I') tel que défini dans la revendication 1 : 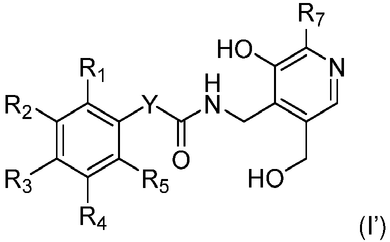 dans laquelle :
R₁, R₂, R₃, R₄, R₅ et R₇, identiques ou différents, représentent chacun un atome d'hydrogène, un atome d'halogène, tel qu'un atome de fluor, de chlore, de brome ou d'iode, un groupement hydroxyle, un radical hydrocarboné linéaire ou ramifié, saturé ou insaturé, en C1-C16, ou encore un radical -OR' où R' représente un radical hydrocarboné linéaire ou ramifié, saturé ou insaturé, en C1-C16,
au moins un groupement parmi R₁, R₂, R₃, R₄ et R₅ représentant un groupement hydroxyle,
et Y représente une liaison covalente ou un radical alcényle en C2-C4,
ou un de ses sels.

La formule générale (I'), de même que toutes les formules présentées ci-après, englobe toutes les combinaisons possibles de formes isomères au niveau des carbones asymétriques, et tous les mélanges de telles formes isomères. A partir d'un mélange d'isomères, chaque isomère particulier peut être obtenu par des méthodes de purification classiques en elles-mêmes pour l'homme du métier.

Par l'expression « véhicule cosmétiquement acceptable », on entend notamment dans la présente, de manière classique en elle-même, que le véhicule est adapté à une utilisation par mise en contact avec des cellules humaines et animales, en particulier les cellules de la peau. De préférence, ce véhicule présente une odeur, une couleur et un toucher agréables, il ne génère pas d'inconforts inacceptables susceptibles de détourner un utilisateur de la composition.

Dans des modes de mise en œuvre particulièrement préférés de l'invention, l'administration de la composition à l'individu est effectuée par application de la composition par voie topique sur la peau de l'individu.

Autrement, l'administration de la composition à l'individu peut notamment être effectuée par voie orale, la présente invention relevant alors plus particulièrement du domaine de la nutraceutique. La composition selon l'invention pour le traitement cosmétique de la peau se présente alors sous la forme d'un complément alimentaire, le véhicule cosmétiquement acceptable étant un véhicule adapté à une administration orale chez l'humain.

Le procédé de traitement cosmétique non-thérapeutique selon l'invention permet avantageusement de prévenir et/ou réparer de manière particulièrement efficace les signes cutanés du vieillissement, par administration, notamment par application par voie topique, de la composition contenant un composé de formule générale (I') ci-dessus sur la peau d'un individu, en particulier sur une zone de peau saine, notamment non atteinte par une maladie inflammatoire.

Ce composé de formule générale (I') permet notamment, et de manière particulièrement efficace, de protéger la peau contre les cassures double-brins de l'ADN induites par des agressions externes telles que les rayons ultraviolets, notamment, de manière tout à fait surprenante, par les UVA, ou l'exposition à la cigarette. Il permet notamment de :
- protéger la peau du stress oxydatif induit par les rayons ultraviolets, plus particulièrement de préserver l'intégrité des cellules cutanées exposées à un rayonnement ultraviolet et de limiter leur vieillissement induit par la surexpression des radicaux libres. A faible concentration, le composé mis en œuvre dans le procédé selon l'invention diminue notamment jusqu'à 40 % la génération de peroxyde d'hydrogène dans les kératinocytes soumis à un stress UV;
- protéger la peau du stress polluant induit par les métaux lourds, notamment le cadmium ;
- protéger la peau du stress polluant induit par la fumée de cigarette, connu notamment pour constituer une agression inductrice de cassures double-brin de l'ADN ;
- de manière plus générale, protéger et régénérer la peau en profondeur. Le procédé selon l'invention permet ainsi de protéger et réparer la barrière épidermique, et de conserver la structure du derme, malgré l'exposition à des agressions externes.

Le composé mis en œuvre dans le procédé selon l'invention présente en outre un effet apaisant, notamment par inhibition de l'expression des cytokines sécrétées lors d'une des premières réponses inflammatoires cutanées, telles que l'Interleukine 8 (IL8) et le facteur de nécrose tumorale (TNFα). En particulier, le composé mis en œuvre dans le procédé selon l'invention réduit l'expression des gènes de l'IL8 et du TNFα par les kératinocytes de 75 % et 45 %, respectivement, et ce même à faible concentration. Il inhibe jusqu'à 30 % la production d'IL8 au sein de kératinocytes soumis à l'inflammation en présence d'interleukine IL1β.

Il a été montré par les présents inventeurs que ces effets de protection / réparation de la peau, en particulier concernant les signes du vieillissement, s'exercent notamment, mais non limitativement, par les effets suivants du composé mis en œuvre dans le procédé selon l'invention : induction de la surexpression de certains gènes des cellules de la peau codant des protéines à effet antivieillissement cutané, et inhibition de l'expression d'autres gènes codant des protéines provoquant des symptômes du vieillissement. L'action de ce composé selon l'invention s'exprime notamment par un renforcement de la fonction barrière de la peau, l'induction de la néosynthèse de protéines essentielles de la matrice extracellulaire cutanée, et l'activation de protéines régénératrices ou détoxifiantes au niveau de la peau.

Plus particulièrement, mais non limitativement, il a été démontré par les présents inventeurs que le composé mis en œuvre dans le procédé selon l'invention présente les effets suivants :
- un effet de protection des protéines de la matrice extracellulaire cutanée (MEC), par inhibition de l'expression des métalloprotéases matricielles (MMP) par les kératinocytes. Les métalloprotéases matricielles, en particulier MMP3, dégradent de nombreux constituants de la matrice extracellulaire cutanée, notamment la fibronectine, la laminine, les collagènes et les protéoglycanes. Le composé mis en œuvre dans le procédé selon l'invention inhibe notamment jusqu'à 50 % l'expression de la MMP3 par les kératinocytes, et jusqu'à 75 % l'expression de la MMP9, autre métalloprotéase matricielle responsable notamment de la dégradation des collagènes ;
- un effet de favorisation de la réparation de l'ADN, notamment par stimulation du gène de la Sirtuine-6 dans les fibroblastes. Localisée dans le noyau de la cellule et associée à la chromatine, cette Sirtuine participe au maintien général de la stabilité du génome, et est impliquée dans la réparation des cassures double-brin, dans la réparation par excisions de base, et dans la protection des télomères. Le composé mis en œuvre dans le procédé selon l'invention stimule jusqu'à 13 % l'expression protéique de la Sirtuine-6, ce qui s'avère particulièrement significatif.

Le composé selon l'invention s'avère notamment plus efficace, en termes de protection / réparation de la peau vis-à-vis des agressions externes, notamment par l'extrait de cigarette, ou l'exposition aux rayonnement ultraviolets, notamment aux UVA, que l'agent antioxydant connu de l'art antérieur qu'est l'acide caféique, de formule : 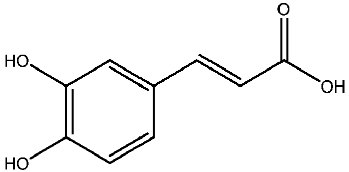 et ce de manière tout à fait inattendue.

Il s'avère de même bien plus efficace que la pyridoxamine, de formule :

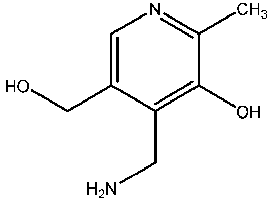

Le procédé selon l'invention met en œuvre un composé de formule générale (I) répondant à la formule plus particulière (I') : 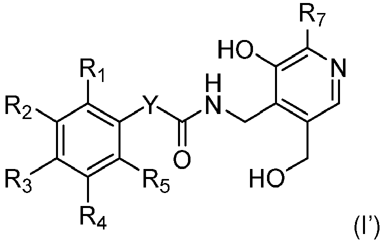 dans laquelle
R₁, R₂, R₃, R₄, R₅ et R₇, identiques ou différents, représentent chacun un atome d'hydrogène, un atome d'halogène, tel qu'un atome de fluor, de chlore, de brome ou d'iode, un groupement hydroxyle, un radical hydrocarboné linéaire ou ramifié, saturé ou insaturé, en C1-C16, ou encore un radical -OR' où R' représente un radical hydrocarboné linéaire ou ramifié, saturé ou insaturé, en C1-C16,
au moins un groupement parmi R₁, R₂, R₃, R₄ et R₅ représentant un groupement hydroxyle,
et Y représente une liaison covalente ou un radical alcényle en C2-C4, de préférence en C2.

Préférentiellement, dans la formule générale (I'), au moins R₁ et R₂ représentent chacun un atome d'hydrogène.

Dans des modes de mise en œuvre particuliers de l'invention, dans la formule (I'), R₃ ne représente pas un atome d'hydrogène et R₁ et R₅ représentent chacun un atome d'hydrogène.

Préférentiellement, dans la formule (I'), au moins un substituant parmi R₂, R₃ et R₄ représente un groupement hydroxyle.

Dans des modes de mise en œuvre particuliers de l'invention, le procédé de traitement cosmétique de la peau met en œuvre un composé de formule générale (I), répondant à la formule plus particulière (I") : 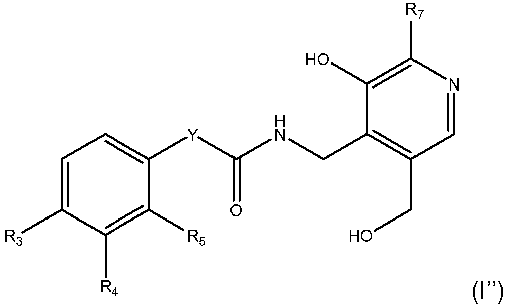 dans laquelle :
R₃, R₄, R₅ représentent chacun un atome d'hydrogène, un atome d'halogène, tel qu'un atome de fluor, de chlore, de brome ou d'iode, un groupement hydroxyle, un radical hydrocarboné linéaire ou ramifié, saturé ou insaturé, en C1-C16, ou encore un radical -OR' où R' représente un radical hydrocarboné linéaire ou ramifié, saturé ou insaturé, en C1-C16,
au moins un premier groupement parmi R₃, R₄ et R₅ représentant un groupement hydroxyle,
et au moins un deuxième groupement parmi R₃, R₄ et R₅ représentant un groupement hydroxyle ou un radical -OR' où R' représente un radical hydrocarboné linéaire ou ramifié, saturé ou insaturé, en C1-C4, de préférence en C1,
R₇ représente un radical hydrocarboné linéaire ou ramifié, saturé ou insaturé, en C1-C4, de préférence en C1,
et Y représente une liaison covalente ou un radical alcényle en C2-C4, de préférence un radical alcényle en C2.

Préférentiellement, dans la formule (I"), R₃ ne représente pas un atome d'hydrogène et R₅ représente un atome d'hydrogène.

Dans la formule (I"), au moins un substituant parmi R₃ et R₄ représente en outre de préférence un groupement hydroxyle.

Dans des modes de mise en œuvre particuliers de l'invention, le procédé de traitement cosmétique de la peau met en œuvre un composé répondant à la formule (I''') : 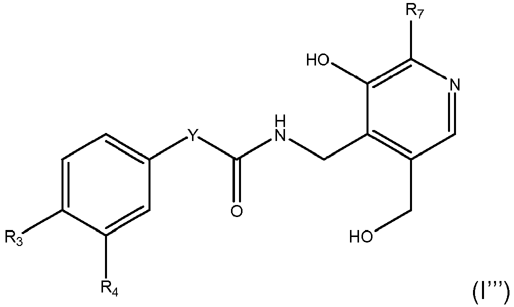 dans laquelle
R₃ représente un groupement hydroxyle, ou un radical -OR' où R' représente un radical hydrocarboné linéaire ou ramifié, saturé ou insaturé, en C1-C16, de préférence un groupement méthyle,
R₄ représente un atome d'hydrogène ou un groupement hydroxyle,
et R₇ représente un radical hydrocarboné linéaire ou ramifié, saturé ou insaturé, en C1-C4, de préférence un groupement méthyle.

Préférentiellement, dans la formule (I'''), R₄ représente un groupement hydroxyle.

Des composés particuliers pouvant avantageusement être mis en œuvre dans le procédé selon l'invention, et présentant une efficacité de protection / réparation cutanée particulièrement importante, notamment par application par voie topique sur la peau d'un individu, répondent aux formules (Ia) à (Ih) ci-après :

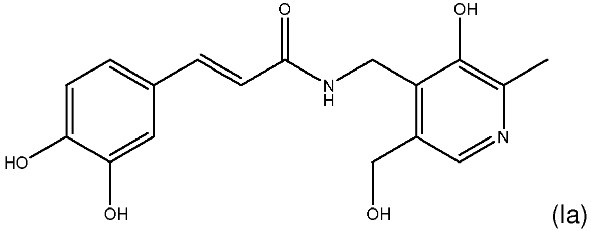

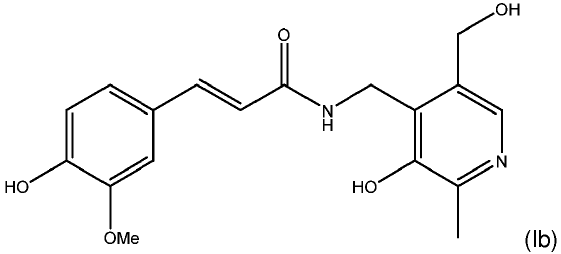

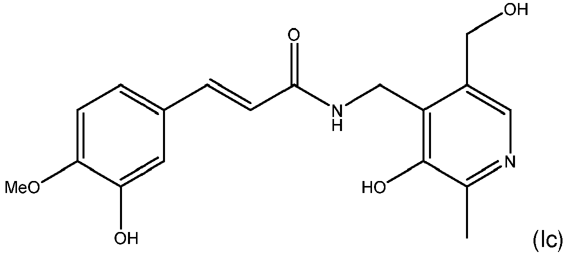

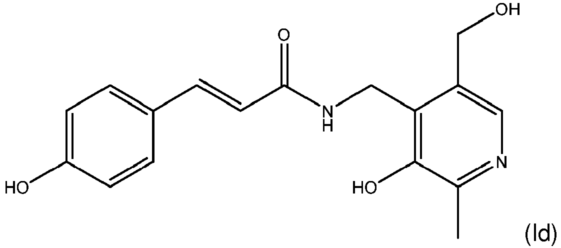

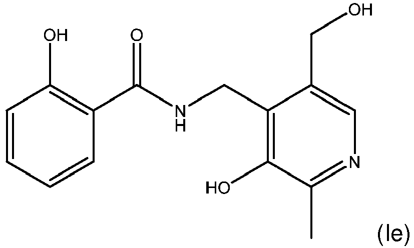

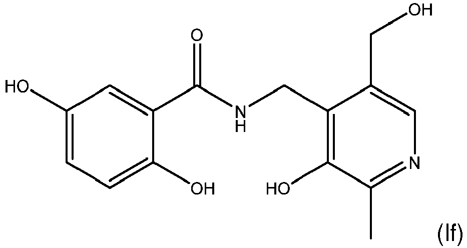

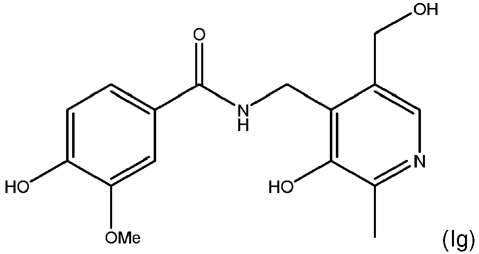

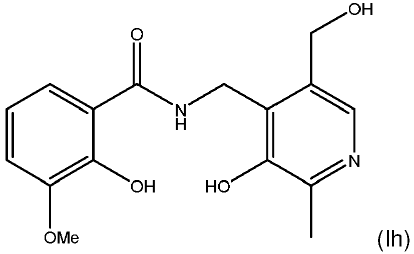

Les composés de formule générale (I') mis en œuvre dans le procédé selon l'invention peuvent être synthétisés par toute méthode connue de l'homme du métier. Ils peuvent notamment aussi bien être synthétisés par voie chimique que par catalyse enzymatique, utilisant par exemple une lipase telle que la lipase B de *Candida antarctica* (CaL-B).

Dans des modes de mise en œuvre particuliers de l'invention, particulièrement avantageux en termes d'efficacité de prévention et de réparation du vieillissement cutané, le composé selon l'invention est présent dans la composition cosmétique à une concentration comprise entre 0,0000001 et 10 % en poids du poids total de la composition, de préférence à une concentration comprise entre 0,00001 et 2 % en poids du poids total de la composition, et préférentiellement comprise entre 0,001 % et 2 % en poids du poids total de la composition.

Plusieurs des composés de formule générale (I') selon l'invention peuvent bien entendu être utilisés simultanément, formulés dans la même composition cosmétique.

La composition mise en œuvre dans le procédé selon l'invention peut se présenter sous toute forme classique en elle-même, notamment, mais non limitativement, sous forme de crème, de pommade, de lait, d'huile, d'onguent, de lotion, de poudre, de solution, de gel, de suspension, de savon, de tampon imbibé, ou encore de shampooing, etc., pour une application par voie topique ; ou sous forme de comprimés, gélules, granulés, poudres, etc., pour une administration par voie orale.

Elle peut en outre comporter tout additif classique en lui-même dans le domaine de la cosmétique, tel qu'un diluant, un agent conservateur, stabilisateur, émulsifiant, adjuvant, transporteur, etc.

L'effet cosmétique recherché peut être renforcé par la mise en œuvre dans la composition de tout autre ingrédient actif supplémentaire, présentant un effet bénéfique pour la peau, cet effet pouvant ou non s'exercer de façon synergique avec celui du composé de formule générale (I') conforme à l'invention. Un tel ingrédient actif supplémentaire peut notamment présenter une activité de réduction des rides, d'augmentation de l'hydratation de la peau, de sa fermeté, de renforcement de sa fonction barrière, d'épaississement de la peau, etc. En tant qu'ingrédient actif pouvant être mis en œuvre en association ou en combinaison avec un composé conforme à l'invention, on peut citer, à titre d'exemple, les agents anti-âges, les agents antirides, les agents desquamants, les agents hydratants, les agents dépigmentants, les agents propigmentants, les agents anti-glycation, les inhibiteurs de NO-synthase, les agents stimulant la synthèse de macromolécules dermiques ou épidermiques et/ou empêchant leur dégradation, les agents stimulant la prolifération des fibroblastes et/ou des kératinocytes ou stimulant la différenciation des kératinocytes, les agents myorelaxants, les agents dermo-décontractants, les agents tenseurs, les agents anti-pollution et/ou anti-radicalaires, les agents anti-irritants, les agents agissant sur la microcirculation, les agents agissant sur le métabolisme énergétique des cellules, les agents anti-UV et leurs mélanges, une telle liste n'étant nullement limitative.

De tels agents peuvent être choisis parmi les caroténoïdes (par exemple beta-carotène, lycopène, astaxanthine, phytoènes), les rétinoïdes (par exemple rétinol, vitamine A, acide rétinoïque cis ou trans, esters de rétinol), les flavanones, les flavonols, les isoflavones (par exemple genisteine, daidzeine, rutine, etc.), les coumarines, les lignanes, les vitamines (par exemple A, B, C, E, F, K, H), les stilbenoïdes, les sapogénines, les acides triterpéniques pentacycliques, les β-hydroxyacides, les hydroxyphénols et leurs dérivés éthers, ester ou hétérosides, les acides phénoliques, les monomères précurseurs des tanins, les aminosucres, les aminoacides (par exemple arginine, lysine, tyrosine, cystéine, taurine, etc.), les peptides (par exemple carnosine, enképhalines, les peptides commerciaux proposés pour leur effet anti-âge tel que la Pal-KTTKS (Matrixyl® de la société Sederma), Ac-Hexapeptide 3 (Argireline de la société Lipotec), Pal-GQPR (Rigin® de la société Sederma), Dermican, Ac-tetrapeptide 9 (Société BASF Beauty Solutions), Syn-ake, tripeptide (Société DMS/Pentapharm), et leurs mélanges, etc.

En particulier, la composition selon l'invention peut contenir, outre le composé de formule générale (I'), un ou plusieurs principes actifs choisis parmi : le rétinol, le resvératrol, l'acide ascorbique, l'acide hyaluronique, le tocophérol, la nicotinamide, l'acide pantothénique, l'Aloe Vera (*Aloe Barbadensis*), l'huile d'Argan (*Argania Spinosa*), le beurre de Karité (*Butyrospermum Parkii*)*,* le calendula (*Calendula Officinalis*), le jojoba (*Buxus Chinensis*), le squalane, la glycérine, la kératine et la caféine, une telle liste n'étant bien entendu nullement limitative de l'invention.

Le procédé de traitement cosmétique de la peau selon l'invention peut notamment comprendre l'application par voie topique, sur la surface cutanée des parties concernées du corps de l'individu en ayant besoin, par exemple sur la surface cutanée du visage, d'une quantité déterminée de la composition à base du composé de formule générale (I') en tant que principe actif, et ce par exemple à raison d'une ou deux fois par jour, par exemple le matin et le soir, et par exemple pendant une période comprise entre 2 semaines et 2 mois ou plus.

Selon un autre aspect, la présente invention concerne l'utilisation d'un composé de formule générale (I') tel que défini ci-avant, ou d'un de ses sels, pour le traitement cosmétique, non-thérapeutique, de la peau d'un individu, en particulier par voie topique, notamment pour la prévention et/ou la réparation des signes du vieillissement cutané, en particulier vis-à-vis d'une exposition à des agressions externes. Ce composé peut à cet effet être compris dans une composition adaptée à une administration à l'humain, en particulier par application topique sur la peau. Ce composé et cette composition, de même que les modalités de cette utilisation, peuvent répondre à l'une ou plusieurs des caractéristiques décrites ci-avant en référence au procédé de traitement cosmétique selon l'invention.

Plus particulièrement, l'invention concerne l'utilisation d'un composé de formule générale (I') tel que défini ci-avant, ou d'un de ses sels, pour, à titre de traitement cosmétique non-thérapeutique de la peau :
- un traitement détoxifiant,
- le traitement du stress oxydatif,
- un traitement des dommages liés à la fumée de cigarette,
- un traitement anti-inflammatoire et/ou apaisant,
- et/ou un traitement des dommages de l'ADN dus aux radicaux libres.

Par traitement, on entend dans la présente description aussi bien la prévention des dommages de la peau, que la réparation / le soin de la peau.

Un autre aspect de l'invention concerne une composition cosmétique, destinée notamment au traitement de la peau, et notamment à la prévention et/ou à la réparation des effets du vieillissement cutané, en particulier liés aux agressions externes, et destinée notamment à une mise en œuvre dans un procédé de traitement cosmétique de la peau selon la présente invention. Cette composition contient un composé de formule générale (I') tel que défini ci-avant, ou un des ses sels, dans un véhicule cosmétiquement acceptable.

Ce composé et cette composition peuvent répondre à l'une ou plusieurs des caractéristiques décrites ci-avant en référence au procédé de traitement cosmétique selon l'invention.

Sont cependant préférentiellement exclus de cette composition, dans le cadre de la présente invention, les composés suivants :
3-(2,4-dihydroxyphényl)-N-(3-hydroxy-5-hydroxyméthyl-2-méthylpyridin-4-ylméthyl)-acrylamide
3-(3,5-dihydroxyphényl)-N-(3-hydroxy-5-hydroxyméthyl-2-méthylpyridin-4-ylméthyl)-acrylamide
3-(2,6-dihydroxyphényl)-N-(3-hydroxy-5-hydroxyméthyl-2-méthylpyridin-4-ylméthyl)-acrylamide

La composition selon l'invention peut notamment contenir un ou plusieurs composés répondant à la formule générale (I"), de préférence un ou plusieurs composés répondant à la formule générale (I'''), et par exemple le composé de formule (la) et/ou le composé de formule (Ic), ces formules étant telles que définies ci-avant. En particulier, la composition selon l'invention peut se présenter sous une forme adaptée à une application par voie topique.

Il a en outre été constaté par les présents inventeurs que les composés de formule générale (I'), répondant à l'une ou plusieurs des caractéristiques ci-avant, et en particulier les composés de formule (I"), et particulièrement les composés de formule (I'''), présentent un effet anti-inflammatoire, lié à leur action décrite ci-avant d'inhibition de l'expression des cytokines sécrétées lors d'une des premières réponses inflammatoires cutanées, telles que l'Interleukine 8 (IL8) et le facteur de nécrose tumorale (TNFα).

Ainsi, selon un autre aspect, la présente invention concerne un composé de formule générale (I'), ou un de ses sels, pour son utilisation thérapeutique pour le traitement d'une maladie inflammatoire de la peau, en particulier par administration par voie topique sur une zone de la peau présentant ladite maladie inflammatoire. Le composé de formule générale (I') peut répondre à l'une ou plusieurs des caractéristiques décrites ci-avant en référence au procédé de traitement cosmétique selon l'invention.

Une méthode de traitement thérapeutique d'une maladie inflammatoire de la peau d'un individu comprend l'administration audit individu, notamment l'application par voie topique sur une zone de la peau dudit individu atteinte de ladite maladie inflammatoire, d'un composé de formule générale (I'), ou un de ses sels. Ce composé de formule générale (I') peut répondre à l'une ou plusieurs des caractéristiques décrites ci-avant en référence au procédé de traitement cosmétique selon l'invention.

L'invention concerne également une composition pharmaceutique / dermatologique comprenant un tel composé, dans un véhicule pharmaceutiquement acceptable. Cette composition, destinée aux humains et/ou aux animaux, peut être avantageusement adaptée pour une administration par voie topique sur la peau d'un individu en ayant besoin.

Cette composition peut répondre à l'une ou plusieurs des caractéristiques décrites ci-avant en référence à la composition cosmétique selon l'invention.

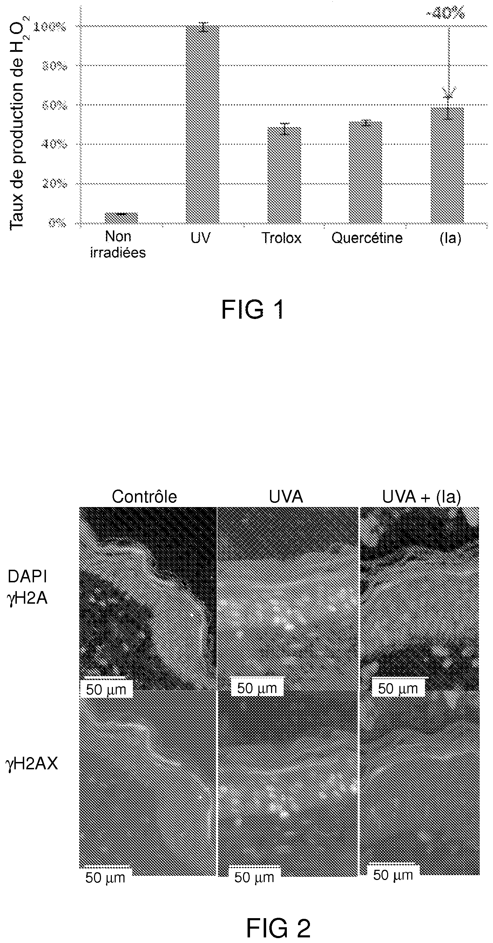

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 7, dans lesquelles :
- la figure 1 représente un graphe en barres montrant le taux de production de H₂O₂ par des kératinocytes humains normaux soumis à un stress aux rayons UVA, sans traitement (UV), ou avec un traitement par du Trolox, de la Quercétine ou par le composé (la) conforme à l'invention, ou pour des cellules non irradiées ;
- la figure 2 montre des coupes observées au microscope d'explants de peau ayant été soumis à des stress aux UVA, traités (UVA + (la)) ou non (UVA) par un composé (la) conforme à l'invention, après marquage γ-H2Ax par immunofluorescence ou coloration au DAPI - le contrôle correspondant à des cellules non soumises à rayons UVA ;

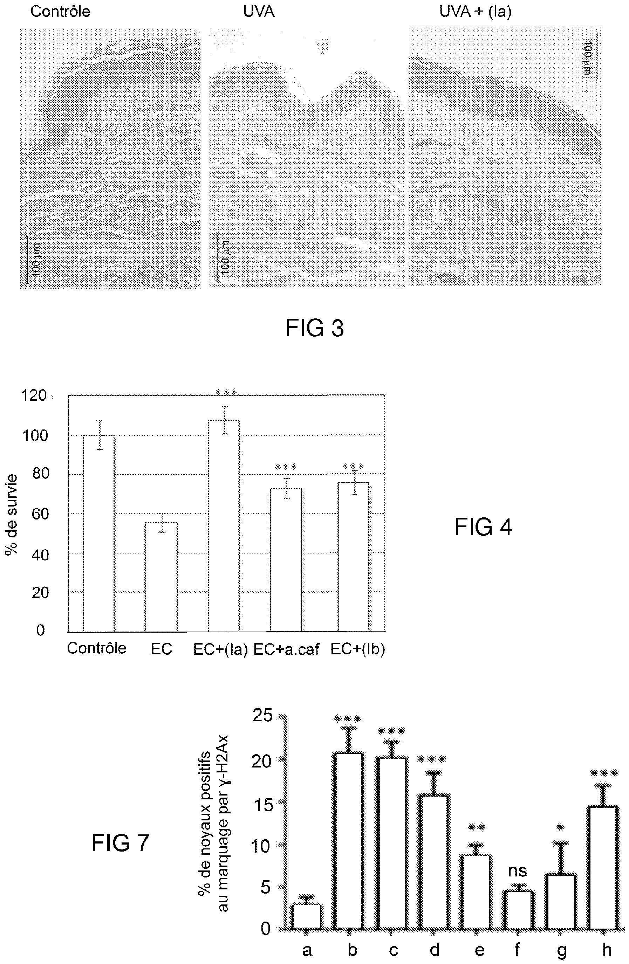

- la figure 3 montre des clichés pris au microscope (grossissement 20x) d'explants de peau ayant été soumis à des stress aux UVA, traités (UVA + (la)) ou non (UVA) par un composé (la) conforme à l'invention, après coloration à l'hématoxyline - le contrôle correspondant à des cellules non soumises à rayons UVA ;
- la figure 4 représente un graphe en barres montrant le pourcentage de survie de kératinocytes NHEK exposés pendant 24 h à un extrait de cigarette, seul (EC) ou avec prétraitement et traitement par des composés selon l'invention (EC+(Ia), EC+(Ib)) ou par l'acide caféique (EC+a.caf), le Contrôle désignant des cellules non traitées et non exposées à l'extrait de cigarette ;

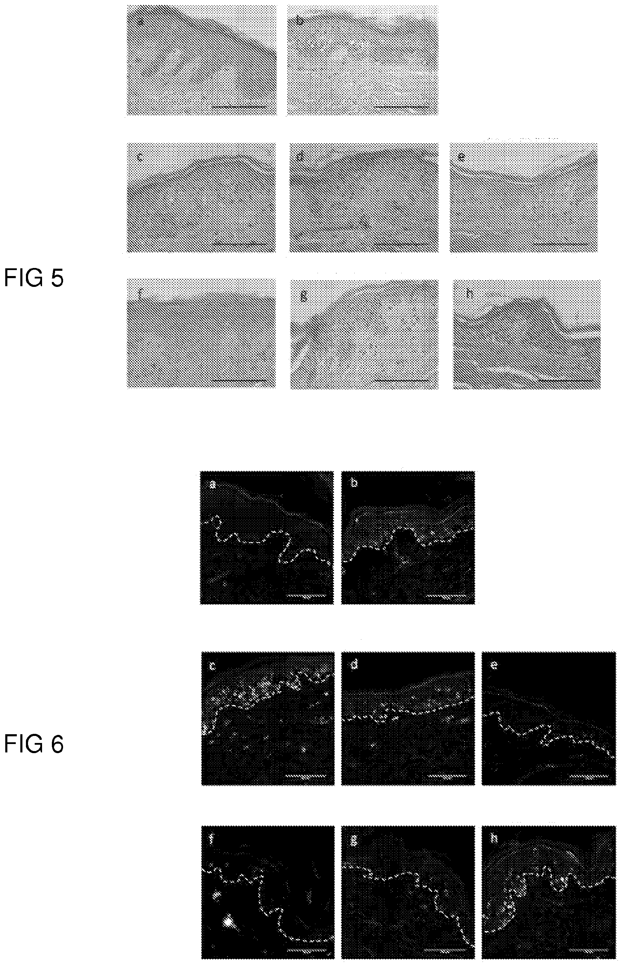

- la figure 5 montre des images de microscopie à fluorescence obtenues sur un explant de peau après marquage à l'hématoxyline et l'éosine, l'explant étant a/ non irradié non traité, b/ irradié aux UVA non traité, c/ irradié aux UVA traité avec l'acide caféique, d/ irradié aux UVA traité avec la pyridoxamine, e/ irradié aux UVA traité avec le composé (la) selon l'invention, f/ irradié aux UVA traité avec le composé (Ic) selon l'invention, g/ irradié aux UVA traité avec le composé (Id) selon l'invention, h/ irradié aux UVA traité avec le composé comparatif Comp.1 ;
- la figure 6 montre des images de microscopie à fluorescence obtenues sur un explant de peau après marquage à γ-H2AX et au 4',6-diamidino-2-phénylindole, l'explant étant a/ non irradié non traité, b/ irradié aux UVA non traité, c/ irradié aux UVA traité avec l'acide caféique, d/ irradié aux UVA traité avec la pyridoxamine, e/ irradié aux UVA traité avec le composé (la) selon l'invention, f/ irradié aux UVA traité avec le composé (Ic) selon l'invention, g/ irradié aux UVA traité avec le composé (Id) selon l'invention, h/ irradié aux UVA traité avec le composé comparatif Comp.1 ;
- et la figure 7 représente un graphe montrant le pourcentage de noyaux positifs au marquage par γ-H2AX quantifié, par rapport au nombre de noyaux totaux, pour des explants de peau respectivement a/ non irradiés non traités, b/ irradiés aux UVA non traités, c/ irradiés aux UVA traités avec l'acide caféique, d/ irradiés aux UVA traités avec la pyridoxamine, e/ irradiés aux UVA traités avec le composé (la) selon l'invention, f/ irradiés aux UVA traités avec le composé (Ic) selon l'invention, g/ irradiés aux UVA traités avec le composé (Id) selon l'invention, h/ irradiés aux UVA traités avec le composé comparatif Comp.1 (moyenne sur 10 échantillons pour chaque condition).

### EXEMPLE 1 - Synthèse chimique de composés conformes à des modes de mise en œuvre particuliers de l'invention

### Protocole général de synthèse

Pour cet Exemple, le protocole de synthèse est le suivant.

Dans un ballon à l'intérieur duquel on a placé un barreau aimanté, sont pesés successivement A g de composant A, D g de composant D puis C g divisé par 2 de composant C. Le ballon est mis sous atmosphère inerte.

On ajoute ensuite F mL de 1,3-dioxolane dans le ballon. Le ballon est alors mis sous agitation magnétique en bain d'huile à 60 °C pendant 30 min.

B g de composant B, C g divisé par 2 de composant C puis G mL de triéthylamine (Et₃N) sont ensuite ajoutés.

Le mélange est alors placé à reflux en bain à 60 °C.

La réaction est suivie sur Chromatographie sur Couche Mince (CCM) (éluant : 7AE/1MeOH/2chloroforme ; révélation à 254 nm ; Rf = 0,28).

Après 3 h de réaction, on laisse la solution revenir à température ambiante.

On ajoute ensuite 100 mL d'acétate d'éthyle (AE) dans le brut réactionnel. On réalise une opération de lavage avec 20 mL de solution aqueuse puis deux opérations d'extraction successives avec 100 mL d'acétate d'éthyle (AE). On récupère la phase organique, que l'on concentre sous pression réduite.

On réalise une purification par chromatographie sur colonne de silice 20-40 µm dite « flash », avec pour éluants : Eluant n°1 : AE ; Eluant n°2 : (1 MeOH/1 ,5chloroforme).

On récupère les fractions d'intérêt que l'on combine et l'on concentre sous pression réduite.

### Composé (Ia)

Le composé conforme à l'invention (E)-N-((3-hydroxy-5-(hydroxyméthyl)-2-méthylpyridin-4-yl)méthyl)-3-(3,4-dihydroxyphényl)acrylamide, de formule (la) : 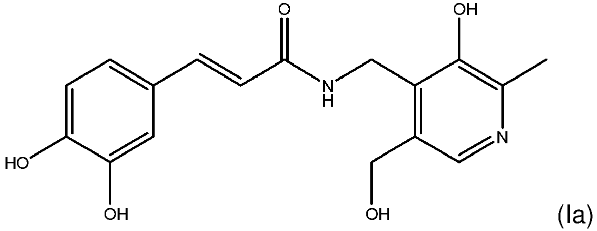
dans laquelle R₁, R₂, R₅ représentent chacun un atome d'hydrogène, R₃ et R₄ représentent chacun un groupement hydroxyle, R₇ représente un groupement méthyle, et Y représente un radical éthylényle,
est préparé comme indiqué dans le protocole général ci-dessus, avec les composants dans les quantités suivantes :
   Composant A : acide caféique / A = 1 g
   Composant B : pyridoxamine dihydrochloride (97 %) / B = 2 g
   Composant C : 1-éthyl-3-(3-diméthylaminopropyl)carbodiimide (EDC.HCl) (98 %) / C = 1,6 g
   Composant D : hydroxybenzotriazole (HOBt) (98 %) / D = 0,9 g
1,3-dioxolane : Volume F = 30 ml
Et₃N : Volume G = 2,81 ml

On récupère finalement 917 mg d'un solide jaune clair (M = 330,34 g.mol⁻¹ ; tr(LCMS) = 3,72 min ; ρ = 50 % ; pureté LC > 98 %).

La méthode d'analyse chromatographique (LCMS) est la suivante : Colonne C18 Gemini NX 50x4,6 mm 5 µm; éluants : eau + 0,1% acide formique (HCOOH) / acétonitrile + 0,1 % HCOOH ; débit 1 ml/min ; début du gradient : 95-5, gradient isocratique pendant 1 minute : 95-5, au bout de 4 min : 0-100, puis gradient isocratique pendant 2 minutes : 0-100, et retour à 95-5 en 1 min. Détection par détecteur UV.

Analyse RMN du proton (¹H ; δ en ppm) CD₃OD : 8.10 (1H), 7.51 (2H, dd, J=15 Hz, J=6Hz), 7.02 (2H, dd, J=6.3Hz, J=1.8Hz), 6.94 (1H, dd, J=4.2Hz, J=2.1 Hz), 6.90(1H, dd, J=4.2Hz, J=2.1 Hz), 6.76 (2H, dd, J=8.1, J=2.7), 6.39 (1H, d, J=15.6Hz), 6.21 (1H, d, J=15.6Hz), 4.60 (2H), 2.60 (3H).

### Composé (Ib)

Le composé conforme à l'invention (E)-3-(4-hydroxy-3-methoxyphenyl)-N-((3-hydroxy-5-(hydroxymethyl)-2-methylpyridin-4-yl)-methyl)-acrylamide, de formule (Ib) : 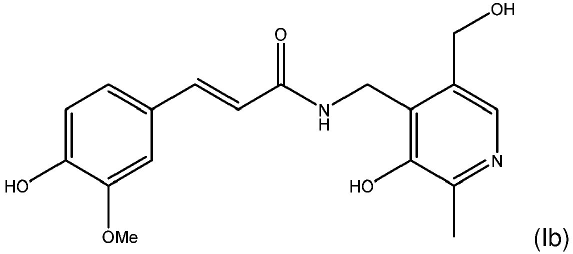
dans laquelle R₁, R₂, R₅ représentent chacun un atome d'hydrogène, R₃ représente un groupement hydroxyle, R₄ représente un groupement -OR' où R' représente un groupement méthyle, R₇ représente un groupement méthyle, et Y représente un radical éthylényle,
est préparé comme indiqué dans le protocole général ci-dessus, avec les composants dans les quantités suivantes :
   Composant A : acide férulique / 1 g
   Composant B : pyridoxamine dihydrochloride (97 %) / 1,9 g
   Composant C: 1-éthyl-3-(3-diméthylaminopropyl)carbodiimide (EDC.HCl) (98 %) / 1,6 g
   Composant D : hydroxybenzotriazole (HOBt) (98 %) / 0,9 g
   1,3-dioxolane : Volume E = 30 ml
   Et₃N : Volume F = 2,81 ml

On récupère finalement 958 mg d'un solide jaune (M = 344,37 g.mol⁻¹ ; tr(LCMS) = 5,78 min ; 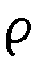 = 54 % ; pureté LC > 90 %).

La méthode d'analyse chromatographique (LCMS) est la suivante : Colonne C18 Gemini NX 150x4,6 mm 5 µm; éluants : eau + 0,1% acide formique (HCOOH) / acétonitrile + 0,1 % HCOOH ; débit 1 ml/min ; début du gradient : 95-5, au bout de 9 min : 5-95, puis gradient isocratique pendant 1 min : 5-95, et retour à 95-5 en 1 min. Détection par détecteur UV.

La méthode d'analyse chromatographique (LCMS) est comme indiqué ci-avant pour le composé (Ia) , mais avec : début du gradient : 95-5, gradient isocratique pendant 1 minute : 95-5, au bout de 4 min : 0-100, puis gradient isocratique pendant 2 minutes : 0-100, et retour à 95-5 en 1 min.

### Composé (Ic)

Le composé conforme à l'invention (E)-3-(3-hydroxy-4-methoxyphenyl)-N-((3-hydroxy-5-(hydroxymethyl)-2-methylpyridin-4-yl)-methyl)-acrylamide, de formule (Ic) : 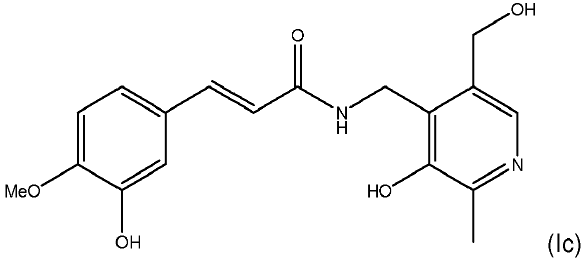
dans laquelle R₁, R₂, R₅ représentent chacun un atome d'hydrogène, R₄ représente un groupement hydroxyle, R₃ représente un groupement -OR' où R' représente un groupement méthyle, R₇ représente un groupement méthyle, et Y représente un radical éthylényle,
est préparé comme indiqué dans le protocole général ci-dessus, avec les composants dans les quantités suivantes :
   Composant A : acide isoférulique / 1 g
   Composant B : pyridoxamine dihydrochloride (97 %) / 1,9 g
   Composant C: 1-éthyl-3-(3-diméthylaminopropyl)carbodiimide (EDC.HCl) (98 %) / 1,6 g
   Composant D : hydroxybenzotriazole (HOBt) (98 %) / 0,9 g
   1,3-dioxolane : Volume E = 30 ml
   Et₃N : Volume F = 2,81 ml

On récupère finalement 1028 mg d'un solide beige clair (M = 344,37 g.mol⁻¹ ; tr(LCMS) = 5,82 min ; 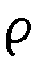 = 58 % ; pureté LC > 99 %).

La méthode d'analyse chromatographique (LCMS) est comme indiqué ci-avant pour le composé (Ia) , mais avec : début du gradient : 95-5, au bout de 9 min : 5-95, puis gradient isocratique pendant 1 min : 5-95, et retour à 95-5 en 1 min.

### Composé (Id)

Le composé conforme à l'invention (E)-N-((3-hydroxy-5-(hydroxymethyl)-2-methylpyridin-4-yl)-methyl)-3-(4-hydroxyphenyl)-acrylamide, de formule (Id) : 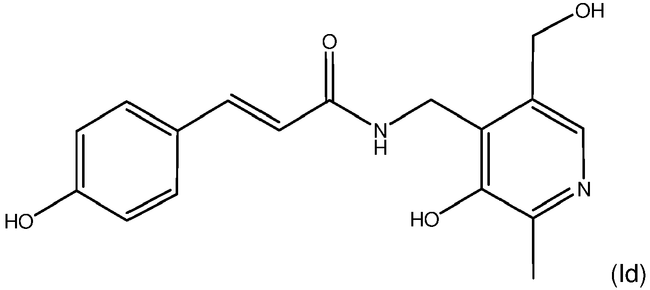
dans laquelle R₁, R₂, R₄, R₅ représentent chacun un atome d'hydrogène, R₃ représente un groupement hydroxyle, R₇ représente un groupement méthyle, et Y représente un radical éthylényle,
est préparé comme indiqué dans le protocole général ci-dessus, avec les composants dans les quantités suivantes :
   Composant A : acide coumarique / 1 g
   Composant B : pyridoxamine dihydrochloride (97 %) / 2,2 g
   Composant C: 1-éthyl-3-(3-diméthylaminopropyl)carbodiimide (EDC.HCl) (98 %) / 1,6 g
   Composant D : hydroxybenzotriazole (HOBt) (98 %) / 0,9 g
   1,3-dioxolane : Volume E = 30 ml
   Et₃N : Volume F = 2,81 ml

On récupère finalement 824 mg d'un solide beige clair (M = 314,34 g.mol⁻¹ ; tr(LCMS) = 5,70 min ; 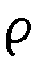 = 43 % ; pureté LC > 99 %).

La méthode d'analyse chromatographique (LCMS) est comme indiqué ci-avant pour le composé (Ia) , avec : début du gradient : 95-5, au bout de 9 min : 5-95, puis gradient isocratique pendant 1 min : 5-95, et retour à 95-5 en 1 min.

Les composés (le) à (Ih) sont synthétisés selon le même mode opératoire que les composés (Ia) à (Id).

### EXEMPLE 2 - Synthèse par catalyse enzymatique du composé (la) conforme à un mode de mise en œuvre particulier de l'invention

La réaction est initiée par l'addition de 360 mg de lipase Novozym 435 dans 100 mL de terbutanol contenant 40 mM d'acide caféique, 100 mM de pyridoxamine dihydrochlorée, et 360 mg de tamis moléculaire activé. La suspension est agitée à 200 rpm pendant 3 jours à 37 °C.

La réaction est suivie sur Chromatographie sur Couche Mince (CCM) (éluant : 7AE/1 MeOH/2chloroforme ; révélation à 254 nm ; Rf = 0,28).

La suspension est ensuite filtrée sur fritté et le filtrat est concentré sous pression réduite.

On réalise une purification par chromatographie sur colonne de silice 20-40 *µ*m dite « flash », avec pour éluants : Eluant n°1 : AE ; Eluant n °2 : (1MeOH/1,5chloroforme).

On récupère les fractions d'intérêt que l'on combine et l'on concentre sous pression réduite.

On récupère finalement 462 mg d'un solide jaune clair (M = 330,34 g.mol⁻¹ ; tr(LCMS) = 3,72 min ; 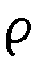 = 35 % ; pureté LC > 98 %).

La méthode d'analyse chromatographique (LCMS) est similaire à celle décrite ci-avant dans l'Exemple 1 en référence au composé (la).

Analyse RMN du proton (¹H ; δ en ppm) CD₃OD : 8.10 (1H), 7.51 (2H, dd, J=15 Hz, J=6Hz), 7.02 (2H, dd, J=6.3Hz, J=1.8Hz), 6.94 (1H, dd, J=4.2Hz, J=2.1 Hz), 6.90(1H, dd, J=4.2Hz, J=2.1 Hz), 6.76 (2H, dd, J=8.1, J=2.7), 6.39 (1H, d, J=15.6Hz), 6.21 (1H, d, J=15.6Hz), 4.60 (2H), 2.60 (3H).

### EXEMPLE 3 - Effet du composé (la) conforme à l'invention sur l'expression de protéines impliquées dans l'inflammation

Cette étude est réalisée sur des Kératinocytes humains normaux (NHEK).

Dans cet exemple, les propriétés anti-inflammatoires du composé (la) sont évaluées par le dosage de l'IL-8, suite à l'application de la cytokine IL-1β à 10 ng/ml sur NHEKs. L'IL-8 est une cytokine inflammatoire majeure produite en première intention lors de la réponse inflammatoire des kératinocytes. La quantification de l'IL-8 dans les surnageants de culture permet une évaluation de l'état inflammatoire des cellules.

L'IL-β est ajoutée au milieu de culture des NHEKs à raison de 10 ng/ml pendant 24h. Après incubation, les milieux de culture sont retirés et remplacés par des milieux de culture contenant le composé (la) à 3 ppm. Après 24 h d'incubation, les surnageants sont récoltés.

L'élément anti-inflammatoire de référence est la dexaméthasone (Sigma) utilisée à 0,4 ppm. Un contrôle ayant subi les mêmes conditions expérimentales sans le composé (Ia) (CTL) ainsi qu'un contrôle non-induit (CTL sans IL-1β) sont également réalisés.

La quantification de l'IL-8 dans 50 µl des surnageants de culture est mesurée par un test ELISA (human CXCL8/IL-8 Immunoassay, R&D Systems).

Chaque traitement est effectué en triplicat.

Les résultats obtenus sont montrés dans le tableau 1.

**Tableau 1 - Effet du composé (la) sur l'expression de protéines impliquées dans l'inflammation de kératinocytes humains normaux (NHEK)**

| Surnageants testés | Quantification de l'IL8 (densité optique) |
|---|---|
| CTL sans IL-1β | 0,06 |
| CTL | 0,23 |
| IL-1β + Dexaméthasone 0,4ppm | 0,13 |
| IL-1β + composé (la) 3ppm | 0,16 |

L'analyse des résultats obtenus montre que le composé (la) conforme à l'invention diminue de manière très hautement significative (p < 0.001) la quantité d'IL-8 produite par les kératinocytes suite à l'induction de l'état inflammatoire par IL-1β, par rapport aux kératinocytes contrôles CTL. Le composé (la) inhibe ainsi de 30% la production d'IL8 au sein des kératinocytes soumis à l'inflammation en présence d'IL-1β.

### EXEMPLE 4 - Effet du composé (la) conforme à l'invention sur la production d'espèces réactives de l'oxygène (ROS) suite à un stress oxydatif UV-induit

Les cellules utilisées pour cet exemple sont des Kératinocytes humains normaux (NHEK).

Les propriétés antioxydantes (neutralisant la production d'espèces réactives de l'oxygène) du composé (la) sont évaluées suite à un stress unique aux UVA sur NHEKs, par un test basé sur l'utilisation de la sonde fluorescente H2DCFDA en triple de culture.

Le H2DCFDA (2'-7'dichlorodihydrofluorescéine diacétate, Fischer Scientific) est une sonde qui, après avoir diffusé dans la cellule et que son groupement acétate ait été clivé par des estérases intracellulaires, peut être oxydée par des radicaux libres (H₂O₂) en DCF (2'-7' dichlorofluorescéine), un composé fluorescent. La production des radicaux libres peut donc être quantifiée par le niveau de fluorescence émis par la sonde oxydée. Plus la cellule produit de radicaux libres, plus le niveau de fluorescence détecté est élevé.

Après 24 heures d'incubation avec le composé (la) (3 ppm dans une solution aqueuse à 0,1% de diméthylsulfoxyde) ou les éléments de référence, les cellules sont mises en présence de la sonde H2DCFDA à 3 ppm pendant 1 heure à 37 °C, 5% CO₂ afin de permettre sa diffusion intracellulaire.

Les éléments antioxydants de référence sont : le Trolox (6-hydroxy-2,5,7,8-tetraméthylchromane, Sigma), à 20 ppm et la Quercétine (quercetin dihydrate, Sigma), à 18 ppm.

Après un rinçage, les cellules sont soumises à un stress unique aux UVA (en l'absence de composé (la)) à raison de 10 joules par centimètre carré. La quantification de la fluorescence émise (excitation : 485 nm ; émission : 520 nm) est effectuée immédiatement après la fin du stress UVA.

Le contrôle (CTL UVA) correspond aux cellules ayant subi le même traitement que celles incubées avec le composé (la), mais sans ce composé. Un contrôle non-exposé aux UVA est également réalisé (CTL sans stress).

Afin de fournir un résultat représentatif de la population cellulaire de chaque condition après un stress unique aux UVA, un test de viabilité cellulaire (MTS) est réalisé immédiatement après la quantification de la fluorescence émise. Les valeurs de fluorescence sont ainsi normalisées par rapport aux viabilités cellulaires afin que les niveaux de fluorescence des différentes conditions puissent être comparés entre eux. Les résultats obtenus sont présentés dans le tableau 2 ci-dessous.

**Tableau 2 - Dosage de la fluorescence au DCFDA après irradiation de aux UVA, pour des cellules NHEKs traitées ou non par le composé (la) conforme à l'invention**

| Surnageants / composés testés | Dosage de la fluorescence au H2DCFDA après irradiation de NHEKs aux UVA |
|---|---|
| CTL sans stress | 16441,9 |
| CTL UVA | 289687,6 |
| UV + Trolox 20 ppm | 269830,3 |
| UV + Quercétine 18 ppm | 207589,6 |
| UV + composé (la) 3 ppm | 198588,8 |

La figure 1 illustre, sous forme de graphe en barres, le taux de production de H₂O₂ pour chaque condition testée.

L'analyse des résultats obtenus montre que le composé (la) diminue de manière très hautement significative (p < 0.001) le niveau d'H₂O₂ intracellulaire produit suite à un stress unique aux UVA. A la faible concentration testée, comme on le voit clairement sur la figure 1, le composé (la) diminue de 40 % la génération d'H₂O₂ intracellulaire dans les cellules en réponse à ce stress.

Le composé (la) conforme à l'invention préserve l'intégrité des cellules cutanées et limite le vieillissement induit par la surexpression de radicaux libres, ce qui démontre ses activités antioxydantes.

### EXEMPLE 5 - Effet de protection du composé (la) conforme à l'invention face au stress polluant

Les cellules utilisées pour cet exemple sont des Fibroblastes humains normaux (NHDF).

Un test de viabilité cellulaire au cadmium est effectué afin de connaitre l'effet de protection du composé (la) face au stress polluant. Le cadmium (Cd) est un métal lourd toxique et écotoxique retrouvé dans l'environnement.

Les cellules sont mises en culture à la concentration de 7000 cellules par puits de plaques 96 puits.

Le prétraitement est effectué 24 heures après l'ensemencement des cellules avec le composé (la) à 10 ppm dans une solution aqueuse à 0,1% de diméthylsulfoxyde, ou sans composé (la) pendant 24 heures.

Après le prétraitement, un traitement au cadmium est effectué. Les cellules sont mises en présence de cadmium à 3 ppm + composé (la) à 10 ppm, ou uniquement en présence de cadmium à 3 ppm (CTL) pendant 24 heures à 37 °C, 5% CO₂. Le contrôle sans stress (CTL sans stress) correspond aux cellules n'ayant pas subi de traitement au cadmium.

Les plaques sont lues au Multiskan® à 450 nm et les résultats obtenus sont présentés dans le tableau 3 ci-dessous.

**Tableau 3 - Viabilité cellulaire suite à une exposition ou non au cadmium de fibroblastes humains normaux, traités ou non par le composé (la) conforme à l'invention**

| Composés testés | Viabilité cellulaire lors de l'exposition ou non au cadmium (densité optique) |
|---|---|
| CTL sans stress | 1,30 |
| CTL (Cd 3 ppm) | 0,95 |
| Cd 3 ppm + composé (la) 10 ppm | 1,28 |

L'analyse des résultats obtenus montre que le composé (la) conforme à l'invention maintient la viabilité cellulaire après une exposition au cadmium, ce qui démontre son effet protecteur de face au stress polluant par les métaux lourds.

### EXEMPLES 6 à 9 - Etude des propriétés du composé (la) conforme à l'invention pour lutter contre les stress externes Induits par les UV

Dans ces exemples, les propriétés de protection du composé (la) contre les dommages causés à l'ADN et à la matrice extracellulaire par une irradiation répétée d'UVA, sont mesurées sur des explants de peau humaine (NATIVESKIN, Genoskin).

Dès la réception des explants, ces derniers sont enduits topiquement de Carbopol® (Lubrizol) + DMSO pour le contrôle non UVA (CTL sans stress) et pour le contrôle avec stress aux UVA (CTL UVA), et de Carbopol® + composé (la) à 100 ppm pour le test.

Sur trois jours, les cellules sont soumises à trois stress aux UVA à raison de 50 joules par centimètre carré.

La première irradiation a lieu 24 heures après les traitements. Les produits sont présents avant, pendant et après l'irradiation. Le contrôle sans stress est enveloppé d'aluminium et également placé dans l'irradiateur.

Lors de l'irradiation, les explants en insert sont disposés dans une boite de pétri avec du PBS. Du milieu frais (Milieu de culture Genoskin) est rajouté après l'irradiation.

Après le troisième jour d'irradiation, les explants sont cultivés 24 heures, puis récoltés et fixés pendant 48 heures en formaline (Sigma). Les échantillons sont inclus en paraffine et coupés.

### Exemple 6 - Protection contre les dommages à l'ADN causés par une irradiation répétée d'UVA par le composé (la) conforme à l'invention

Un marquage γ-H2Ax par immunofluorescence sur coupe de paraffine est effectué, afin de repérer les cassures double-brin de l'ADN.

Les échantillons sont préparés pour le marquage fluorescent. Après déparaffinage, les échantillons sont démasqués par un bain de citrate. Après rinçage et blocage, les échantillons sont incubés en présence de l'anticorps primaire, le γ-H2Ax (Cell Signaling Technology), puis incubés en présence de l'anticorps secondaire (Invitrogen) lié à la fluorescéine durant 1 heure. Après lavage, les échantillons sont ensuite mis en présence de DAPI pendant 10 min afin de marquer les noyaux, puis rincés une nouvelle fois.

Les coupes sont montées sur lame, et observées à l'aide d'un microscope (Leica DM5000B). Les images obtenues sont montrées sur la figure 2. On observe clairement une absence de fluorescence pour l'échantillon traité par le composé (la) selon l'invention, ce qui traduit une basence de cassures double-brin de l'ADN.

La fluorescence est quantifiée au moyen du microscope. Les résultats obtenus sont présentés dans le tableau 4 ci-dessous.

**Tableau 4 - Analyse de l'effet du composé (la) sur la diminution des cassures double brin de l'ADN induites par les UVA**

| Composés testés | Cellules positives au marquage γ-H2Ax rapportées à 100 % / au nombre de cellules total |
|---|---|
| CTL sans stress | 4,34% - 47 / 1116 |
| CTL UVA | 33,93% - 381/1123 |
| Composé (la) 100 ppm | 4,76% - 59/1282 |

L'analyse des résultats obtenus montre une diminution très hautement significative du nombre de cellules positives au marquage γ-H2Ax en présence du composé (la), par rapport à l'explant irradié (p < 0.001, Test t de Welch).

Cette étude démontre l'efficacité du composé (la) conforme à l'invention dans la protection contre les dommages à l'ADN causés par une irradiation répétée d'UVA, le nombre de cellules positives au marquage γ-H2Ax étant alors extrêmement réduit, sensiblement égal à celui des cellules non irradiées.

### Exemple 7 - Effet de protection et de régénération en profondeur de la peau par le composé (la) conforme à l'invention

Une étude morphologique de la peau est mise en œuvre.

Les échantillons d'explant sont déparaffinés, réhydratés puis ils sont soumis à une coloration à l'hématoxyline et à l'éosine afin d'observer l'effet du composé (la) en profondeur sur la peau. Les lames sont ensuite déshydratées.

Le microscope Leica DM5000B au grossissement X20 est utilisé pour faire les observations en 2D. Les résultats sont montrés sur la figure 3.

On y observe que le traitement UVA induit une diminution de l'épaisseur de l'épiderme, une désorganisation des couches épidermique ainsi qu'une accumulation de cornéocytes.

Le composé (la) conforme à l'invention protège et répare : la barrière épidermique est préservée de la dégradation des UVA et la structure du derme est conservée.

### Exemple 8 - Effet du composé (la) conforme à l'invention sur l'expression de la protéine MMP1 (Matrice-Métalloprotéase de type 1)

Un marquage de la protéine MMP1 par immunofluorescence sur coupe de paraffine est effectué.

Les échantillons sont préparés pour le marquage fluorescent. Après déparaffinage, les échantillons sont démasqués par un bain de citrate. Après rinçage et blocage, les échantillons sont incubés en présence de l'anticorps primaire MMP1 (Abcam) puis incubés en présence de l'anticorps secondaire (Invitrogen) durant 1 heure. Après lavage, les échantillons sont ensuite mis en présence de DAPI pendant 10 min afin de marquer les noyaux, puis rincés une nouvelle fois.

Les coupes sont montées sur lame et la fluorescence est quantifiée à l'aide d'un microscope (Leica DM5000B). Les résultats obtenus sont présentés dans le tableau 5 ci-dessous.

**Tableau 5 - Expression de MMP1 dans les cellules traitées ou non par le composé (la) selon l'invention**

| Composés testés | Cellules positives à MMP1 rapportées à 100 % - Intensité moyenne de fluorescence du marquage MMP1 (UA) |
|---|---|
| CTL sans stress | - 141,6 |
| CTL UVA | +16,7 % - 165,2 |
| Composé (la) 100 ppm | +3,2 % - 146,2 |

On observe une augmentation du marquage dans le derme avec l'irradiation UVA (p < 0.001). Après le traitement au composé (la), l'expression de la protéine est similaire à celle obtenue pour le contrôle sans stress, et ce de manière significative (p < 0.05).

Cette étude permet de démontrer l'efficacité du composé (la) conforme à l'invention dans la protection contre les dommages de la matrice extracellulaire causés par une irradiation répétée d'UVA avec le maintien de l'expression de la protéine MMP-1.

### Exemple 9 - Effet du composé (la) conforme à l'invention sur l'expression de la protéine ELN (élastine)

Un marquage de la protéine élastine est effectué par immunofluorescence sur coupe de paraffine.

Les échantillons sont préparés pour le marquage fluorescent. Après déparaffinage, les échantillons sont démasqués par un bain de citrate. Après rinçage et blocage, les échantillons sont incubés en présence de l'anticorps primaire ELN (Abcam) puis incubés en présence de l'anticorps secondaire (Invitrogen) durant 1 heure. Après lavage, les échantillons sont ensuite mis en présence de DAPI pendant 10 min afin de marquer les noyaux, puis rincés une nouvelle fois.

Les coupes sont montées sur lame et la fluorescence est quantifiée à l'aide d'un microscope (Leica DM5000B). Les résultats obtenus sont présentés dans le tableau 6 ci-dessous.

**Tableau 6 - Expression de l'élastine dans les cellules traitées ou non par le composé (la) selon l'invention**

| Composés testés | Cellules positives à ELN rapportées à 100% - Intensité moyenne de fluorescence du marquage ELN (UA) |
|---|---|
| CTL sans stress | - 160,4 |
| CTL UVA | -9% - 145,9 |
| Composé (la) 100 ppm | + 14% - 182,4 |

Cette étude démontre l'efficacité du composé (la) conforme à l'invention dans la protection contre les dommages de la matrice extracellulaire causé par une irradiation répétée d'UVA, avec le maintien de l'expression de la protéine élastine. On obtient également un effet de préservation de la peau du phénomène de sénescence.

### EXEMPLE 10 - Effet du composé (la) conforme à l'invention sur l'expression protéique de la Sirtuine-6

Les cellules utilisées pour cet exemple sont des Fibroblastes humains normaux (NHDF).

Dans cet exemple, l'expression de la Sirtuine-6 est mesurée au niveau protéique par la technique d'immunofluorescence, après incubation *in vitro* des cellules en absence ou en présence de composé (la) à une concentration de 10 ppm dans une solution aqueuse à 0,1% de diméthylsulfoxyde (DMSO).

Les cellules sont mises en culture à la concentration de 5000 cellules par puits de plaques 96 puits, en présence de milieu de culture standard pour NHDF.

La mise en contact avec le composé (la) est effectuée pendant 24 heures.

Le TGFβ est utilisé comme témoin positif à la concentration de 10 ng/ml dans une solution aqueuse. Un contrôle non traité est également réalisé.

Le surnageant est éliminé et les cellules sont préparées pour le marquage fluorescent. Les cellules sont fixées en présence de formaldéhyde 3,7 %, puis perméabilisées dans un tampon contenant 1% de Triton®, enfin les cellules sont rincées et incubées en présence d'un anticorps anti-SIRT6 (Abcam) pendant 2 heures. Après lavage, les cellules sont incubées en présence de l'anticorps secondaire (Alexa GAR488, Invitrogen) durant 1 h. Après lavage, les cellules sont incubées en présence de DAPI pendant 10 min, puis rincées une nouvelle fois.

La fluorescence est ensuite quantifiée à l'aide d'un microscope automatisé (ArrayScan® (Cellomics®)).

Le résultat obtenu, en terme d'expression de la Sirtuine-6 par rapport au niveau basal, est présenté dans le tableau 7 ci-dessous.

**Tableau 7 - Effets du composé (la) sur l'expression protéique de la Sirtuine-6 dans des cellules NHDF**

| Composés testés | Expression protéique de la Sirtuine-6 par rapport au niveau basal rapporté à 100 % |
|---|---|
| Basal | 100 % |
| Composé (la) 10ppm | 113,69 % |

On constate que le composé (la) conforme à l'invention stimule l'expression protéique de la Sirtuine-6 de manière très hautement significative (p < 0.001). L'expression de la Sirtuine-6 est très forte dans le noyau des cellules, signe que la fluorescence est élevée et que le marquage est fort. Une telle augmentation représente donc une activation considérable de l'expression de la protéine. La Sirtuine-6 étant localisée dans le noyau de la cellule et associée à la chromatine, participant au maintien général de la stabilité du génome, et étant impliquée dans la réparation des cassures double-brin de l'ADN, dans la réparation par excision de base et dans la protection des télomères, ces résultats démontrent que le composé (la) conforme à l'invention favorise la réparation de l'ADN.

Il a par ailleurs été démontré par les présents inventeurs que le composé (la) conforme à l'invention stimule l'expression du gène de la Sirtuine-6 de 66 % par rapport au contrôle non traité (voir l'Exemple 13 ci-après).

### EXEMPLES 11 et 12 - Etude de l'effet du composé (la) conforme à l'invention sur l'expression de gènes de cellules cutanées

Dans ces exemples, l'expression des ARN messagers est mesurée par la technique de RT-PCR (transcription inverse suivie de réaction de polymérisation en chaîne) quantitative, après incubation in vitro des différents types cellulaires en absence ou en présence de composé (la).

Cette étude est réalisée sur Fibroblastes dermiques humains normaux (NHDF) ou Kératinocytes épidermiques humains normaux (NHEK). Le composé (la) est testé à 10⁻⁵ M dans une solution aqueuse à 0,1% de diméthylsulfoxyde.

Les cellules sont mises en culture à la concentration de 10 000 cellules par puits de plaques 96 puits, en présence de milieu de culture standard en fonction du type cellulaire.

Le composé (la) est ensuite ajouté à la concentration (3 ppm), pendant 24 heures.

Le surnageant est éliminé et les cellules sont reprises dans un tampon spécifique pour l'extraction des ARN messagers (ARNm). Les ARNm sont purifiés et réverse-transcrits en présence d'une réverse transcriptase commerciale.

Les ADN complémentaires (ADNc) obtenus sont quantifiés par RT-PCR, au moyen d'amorces adéquates. Le taux d'expression des ARNm est normalisé avec 5 gènes de référence.

### Exemple 11 - Effet du composé (la) conforme à l'invention sur l'expression de gènes codant des protéines de la matrice extracellulaire (Matrice-Métalloprotéase de type 3 et de type 9)

Les cellules utilisées pour cet exemple sont des kératinocytes humains normaux (NHEK).

Les résultats obtenus, en termes de diminution de l'expression de MMP-3 et MMP-9 par rapport au niveau basal (absence de composé), sont montrés sur le tableau 8 ci-après.

**Tableau 8 - Effet du composé (la) sur l'expression de MMP-3 et MMP-9 par des cellules NHEK traitées ou non par le composé (la)**

| Gènes testés | Diminution de l'expression génique par rapport au niveau basal rapporté à 100 % |
|---|---|
| MMP-3 | - 50 % |
| MMP-9 | - 75 % |

Il en ressort que le composé (la) protège fortement les protéines de la matrice extracellulaire cutanée, et permet une diminution du niveau d'expression des protéines MMP-3 et MMP-9 par les kératinocytes. Une telle diminution induit un ralentissement de la dégradation de nombreux constituants de la matrice extracellulaire (Fibronectine, Laminine, Collagènes et Protéoglycanes).

### Exemple 12 - Effet du composé (la) conforme à l'invention sur l'expression génique de TNFα (facteur de nécrose tumorale) et IL8 (Interleukine 8)

Les cellules utilisées pour cet exemple sont des kératinocytes humains normaux (NHEK).

Les résultats obtenus, en termes de diminution de l'expression de TNFα et IL8 par rapport au niveau basal (absence de composé), sont montrés sur le tableau 9 ci-après.

**Tableau 9 - Effet du composé (la) sur l'expression TNFα et IL8 par des cellules NHEK traitées ou non par le composé (la)**

| Gènes testés | Diminution de l'expression génique par rapport au niveau basal rapporté à 100 % |
|---|---|
| TNFα | - 45 % |
| IL8 | - 75 % |

Il en ressort qu'à la faible concentration testée, le composé (la) réduit l'expression des gènes TNFα et IL8 de manière significative, ce qui favorise l'apaisement de la peau.

### EXEMPLE 13 - Effet du composé (la) conforme à l'invention sur l'expression génique de la Sirtuine-6

Localisée dans le noyau de la cellule et associée à la chromatine, la Sirtuine-6 participe au maintien général de la stabilité du génome. Elle est impliquée dans la réparation des cassures double brin, dans la réparation par excision de base et dans la protection des télomères.

Les cellules utilisées pour cet exemple sont des Fibroblastes humains normaux (NHDF). Les cellules sont ensemencées dans des plaques 96 puits à une concentration de 10000 cellules par puits.

La mise en contact avec le composé testé est effectuée pendant 24 h. Après la mise en contact 24h, le surnageant est éliminé et les cellules sont collectées dans une solution de lyse pour extraire les ARNm. Les ARNm sont reverse-transcrits en ADN puis quantifiés par PCR quantitative en temps réel.

Les résultats obtenus, en termes d'expression de la Sirtuine-6 par rapport au niveau basal (absence de composé) rapporté à 100 %, sont montrés sur le tableau 10 ci-après.

**Tableau 10 - Effet du composé (la) sur l'expression génique de la Sirtuine-6 dans des cellules NHDF**

| Composés testés | Expression génique de la sirtuine-6 par rapport au niveau basal rapporté à 100 % |
|---|---|
| Basal | 100 % |
| Composé (la) | 166 % |

Une augmentation significative du niveau d'expression de la Sirtuine-6 est observée après mise en contact avec le composé (la) conforme à l'invention, ce qui induit que ce composé stimule le gène de la Sirtuine-6, et favorise la réparation de l'ADN.

### EXEMPLE 14 - Effet protecteur et/ou réparateur du composé (la) conforme à l'invention suite aux dommages induits par un extrait de cigarette.

Les cellules utilisées pour cet exemple sont des Fibroblastes humains normaux (NHDF) et des Kératinocytes épidermiques humains normaux (NHEK).

Un extrait de cigarette est préparé à l'aide d'un dispositif de laboratoire permettant l'aspiration de la fumée de cigarette grâce à un système d'aspiration, et le bullage contrôlé de cette fumée de cigarette dans la solution étudiée, qui est ensuite filtrée sur un filtre en Polyéther sulfone (PES) de 0,22 µm.

Les cellules sont mises en culture à la concentration de 7000 cellules par puits de plaques 96 puits (NHDF) ou 8000 cellules par puits (NHEK), en présence de milieu de culture standard pour NHDF ou NHEK (pendant 24 h, 37 °C, 5% CO₂).

Après prétraitement des cellules avec le composé (la) à 10 ppm pendant 24 h, les cellules sont traitées avec l'extrait de cigarette dilué au 1/50^{ème} dans le milieu de culture avec ou sans composé (la) à 10 ppm pendant 24 h. L'absorbance est lue une première fois à 450 nM (Multiskan®).

Ensuite, 10 µl de WST1 (Roche) sont introduits dans le milieu et les cellules sont incubées pendant 3 heures.

L'absorbance à 450 nm est mesurée après l'addition de WST1 (Multiskan®) et les résultats obtenus, en termes de viabilité cellulaire, sont montrés sur les tableaux 11 et 12 ci-après. Les cellules traitées (Extrait de cigarette + (la)) sont comparées aux cellules non traitées (Contrôle) ou aux cellules ayant uniquement subi une exposition à l'extrait de cigarette (Extrait de cigarette).

**Tableau 11 - Cytotoxicité de l'extrait de cigarette sur des cellules NHDF avec ou sans traitement par le composé (la)**

| Composés testés | Viabilité cellulaire sur NHDF (Densité optique) |
|---|---|
| Contrôle | 1,618 |
| Extrait de cigarette | 1,357 |
| Extrait de cigarette + (la) 10 ppm | 1,674 |

**Tableau 12 - Cytotoxicité de l'extrait de cigarette sur des cellules NHEK avec ou sans traitement par le composé (la)**

| Composés testés | Viabilité cellulaire sur NHEK (Densité optique) |
|---|---|
| Contrôle | 2,070 |
| Extrait de cigarette | 1,392 |
| Extrait de cigarette + (la) 10 ppm | 2,589 |

L'analyse des résultats obtenus montre que le composé (la) maintient la viabilité cellulaire en présence d'extrait de cigarette (dilué au 1/50°), ce qui démontre l'effet protecteur de ce composé de manière respectivement hautement (p < 0.01) et très hautement significative (p < 0.001) sur les cellules NHEK et NHDF.

### EXEMPLE 15 - Effet du composé (la) conforme à l'invention sur les dommages à l'ADN (cassures doubles-brins) induits par un extrait de cigarette

Les cellules utilisées pour cet exemple sont des Fibroblastes humains normaux (NHDF) et des Kératinocytes épidermiques humains normaux (NHEK).

Les cellules sont mises en culture à la concentration de 3000 cellules par puits de plaques 96 puits en présence de milieu de culture standard pour NHDF ou NHEK (pendant 24 heures, 37 °C, 5% CO₂).

Après prétraitement des cellules avec le composé (la) à 10 ppm pendant 24 heures, les cellules sont avec l'extrait de cigarette dilué au 1/50° avec ou sans composé (la) à 10 ppm pendant 24 heures. L'absorbance est lue une première fois à 450nM (Multiskan®).

Après lavage, fixation en formaline et perméabilisation des cellules, ces dernières sont incubées avec des anticorps ciblant γ-H2AX (Millipore) et révélées par un anticorps secondaire (Invitrogen) lié à la fluorescéine durant 45 min.

Après lavage, les échantillons sont mis en présence de DAPI pendant 10 min afin de marquer les noyaux, puis rincés une nouvelle fois. Les protéines marquées sont observées et quantifiées au microscope automatisé à fluorescence (ArrayScan® (Cellomics®)). Les cellules traitées par le composé (la) sont ensuite comparées aux cellules non traitées ou aux cellules uniquement exposées à l'extrait de cigarette.

Les résultats obtenus sont montrés sur les tableaux 13 et 14 ci-après.

**Tableau 13 - Cassures double-brin de l'ADN induites par un extrait de cigarette sur des cellules NHDF avec ou sans traitement par le composé (la)**

| Composés testés | Cassures double-brin de l'ADN sur NHDF (UA) |
|---|---|
| Contrôle | 6441 |
| Extrait de cigarette | 11404*** |
| Extrait de cigarette + (la) 10 ppm | 8752*** |

**Tableau 14 - Cassures double brin de l'ADN induites par un extrait de cigarette sur des cellules NHEK avec ou sans traitement par le composé (la) (*** p<0,001 et * p<0.05, test non paramétrique de Mann-Whitney)**

| Composés testés | Cassures double-brin de l'ADN sur NHEK (UA) |
|---|---|
| Contrôle | 10346 |
| Extrait de cigarette | 13269*** |
| Extrait de cigarette + (la) 10 ppm | 11193* |

L'analyse des résultats obtenus montre que le composé (la) conforme à l'invention est capable de neutraliser le stress induit par l'extrait de cigarette en réduisant de manière significative la quantité de γH2AX dans NHDF et NHEK.

Cette étude démontre l'efficacité du composé (la) dans la protection contre le stress induit par l'extrait de cigarettes en empêchant ou en favorisant la réparation des dommages doubles d'ADN. Ces résultats confirment l'action détoxifiante et de préventif anti-âge du composé (la) conforme à l'invention.

### EXEMPLE 16 - Effet sur la diminution de la survie de kératinocytes humains induite par un extrait de cigarette, des composés conformes à l'invention (la) et (Ib), et de l'acide caféique à titre comparatif

Cette étude vise à comparer l'effet protecteur ou réparateur des composés conformes à l'invention (la) et (Ib) vis-à-vis des dommages induits sur la peau par un extrait de cigarette. A titre comparatif, l'acide caféique (fournisseur : Acros) est également testé. Cet acide constitue un motif de base des composés (la) et (Ib) conformes à l'invention

Les cellules NHEK sont ensemencées dans une plaque à 96 puits dans un milieu approprié (Promocell), à raison de 9000 cellules par puits, et incubées 24 h à 37 °C et 5 % dioxyde de carbone.

Les composés à tester sont préparés comme suit :
- (Ia) : 10 mg de composé + 909 µL de DMSO
- (Ib) : 7 mg de composé + 636 µL de DMSO
- acide caféique : 10 mg de composé + 909 µL de DMSO

Chaque solution mère ci-dessus est diluée 1000 fois, jusqu'à 10 ppm dans le milieu de culture, puis les solutions obtenues sont filtrées au moyen d'un filtre de nylon (0,22 µm). Les solutions finales sont concentrées à 10 ppm dans 0,1 % de DMSO.

Des solutions à 20 ppm dans 0,2 % de DMSO sont également préparées de manière similaire, pour dilution dans l'extrait de cigarette.

Il est préparé un extrait de 20 cigarettes, comme indiqué dans l'exemple 14 ci-avant. Cet extrait est ensuite dilué à 1/50 dans le milieu de culture ; ou à 2/50 dans le milieu de culture, puis à 1/2 dans le milieu de culture, auquel le composé à tester est ajouté à 20 ppm.

Les cellules sont prétraitées pendant 24 h avec le composé à tester à 10 ppm dans le milieu de culture.

Les cellules sont ensuite traitées avec l'extrait de cigarette dilué au 1/50^{ème} avec ou sans le composé testé à 10 ppm pendant 24 h.

3 h avant la fin du test, 10 µl de réactif de prolifération cellulaire WST1 (Roche) sont ajoutés dans chaque puits. L'absorbance à 450 nm est mesurée avant et après l'addition de réactif WST1, au moyen d'un lecteur Multiskan®. Les cellules traitées sont comparées avec les cellules non traitées et avec les cellules exposées à l'extrait de cigarette.

Les résultats obtenus, en termes de % de survie des cellules pour chaque condition testée, sont montrés sur la figure 4. On y observe que l'extrait de cigarette (EC) réduit fortement le pourcentage de survie des cellules (jusqu'à 55 % de survie). L'acide caféique a un léger effet sur la survie des cellules (73 % de survie). Le composé (Ib) selon l'invention a également un léger, mais significatif, effet d'amélioration de la survie des cellules (76 % de survie). Le composé (la) selon l'invention a quant à lui un très fort effet de protection des cellules exposées à l'extrait de cigarette (108 % de survie des cellules).

Ainsi, le composé (la) selon l'invention protège totalement les cellules contre le stress induit par l'extrait de cigarette, et notamment, et de manière très surprenante, bien mieux que l'acide caféique qui constitue un motif de sa structure.

### EXEMPLE 17 - Evaluation de l'efficacité protectrice de composés conformes à l'invention (la), (Ic) et (Id) et de composés comparatifs contre les UVA sur explant de peau ex-vivo

Les composés testés sont les suivants :
- composés selon l'invention (la), (Ic) et (Id),
- acide caféique,
- pyridoxamine,
- et composé comparatif Comp.1, de formule :

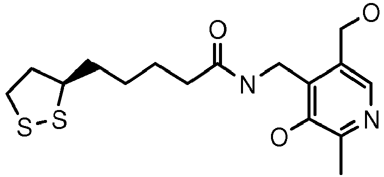

### 1/ Protocole

### 1.1 / Traitement des échantillons

Le test a été mené sur explants de peau NativeSkin® (Genoskin), une biopsie de peau baignée dans une matrice solide et nourrissante tandis que sa surface épidermique est conservée en contact avec l'air. La biopsie est fermement ancrée dans la matrice et scellée pour empêcher toute diffusion latérale de formulation destinée à une application topique.

L'étude a été menée sur un donneur de 51 ans (phototype 2) dans les conditions suivantes.

Les composés testés sont mis en œuvre dans une composition les contenant à 0,9 mmol/L dans un mélange de Carbopol® (0,5 % p/p), DMSO (0,1 % p/p) et H₂O (qsp 100%).

Les échantillons sont irradiés à 60J/cm² UVA tous les jours pendant 3 jours. On effectue un traitement durant 24h avant l'irradiation, par application de la composition testée sur l'échantillon (dépôt de 40 µl de composition sur la couche cornée), puis un post-traitement de 24h après l'irradiation, de la même façon.

On réalise également un Contrôle d'échantillon de peau non irradié et un Contrôle d'échantillon de peau irradié, tous deux avec application du seul véhicule (0,5 % Carbopol®, 0,1 % DMSO, H₂O). Le Contrôle d'échantillon de peau non irradié est introduit dans l'irradiateur dans une feuille d'aluminium, de sorte à le protéger contre les UV.

Les échantillons sont prélevés, coupés (selon la méthode classique de cryo-coupe mettant en œuvre paraffine et congélation) et fixés dans 1 ml de formaline pendant 24 h.

### 1.2/ Méthode de détection

Les explants sont ensuite inclus en paraffine et des sections de 5µm sont réalisées.

Les lames sont ensuite colorées avec de l'hématoxyline (composé basique marquant les noyaux) et de l'éosine (composé acide marquant les tissus conjonctifs).

Les lames obtenues sont aussi marquées par immunohistochimie avec le biomarqueur γ-H2Ax.

L'analyse des marquages est réalisée au microscope à fluorescence DM5000B (Leica Microsystems) et 10 images sont acquises pour chaque échantillon.

### 1.3/ Analyse des données

Pour le marquage avec γ-H2Ax, pour chaque image, on détecte avec du 4',6-diamidino-2-phénylindole (dapi) le nombre de noyaux totaux. Sur la même image, on quantifie le nombre de noyaux présentant le marquage. On effectue le rapport du nombre de noyaux positifs, ramené à 100, sur le nombre de noyaux totaux, pour obtenir un pourcentage (= (noyaux positifs*100)/noyaux totaux), n=10 par échantillon.

On effectue une analyse statistique avec un test de student en comparant les échantillons au témoin (valeur p < 0.5*, valeur p < 0.05**, valeur p < 0.005***, ns non significatif).

### 2/ analyse histologique

Les images obtenues par microscope à fluorescence sont montrées sur la figure 5.

Comme on peut le voir sur cette figure, l'échantillon contrôle (a) présente une morphologie de l'épiderme classique avec la stratification des kératinocytes en 4 couches cohésives.

L'échantillon de peau irradié (b) présente une modification de l'organisation des couches de l'épiderme ainsi que l'apparition de noyaux pycnotiques révélant un phénomène d'apoptose. On retrouve le phénotype de l'échantillon irradié pour les peaux irradiées et traitées par l'acide caféique (c), la pyridoxamine (d) et le composé comparatif Comp.1 (h).

La peau semble partiellement protégée avec le composé (Id) selon l'invention (g).

Avec les composés (la) (e) et (Ic) (f) selon l'invention, la morphologie de l'épiderme est identique à celle du contrôle non irradié. La peau est totalement protégée des effets délétères des UVA.

### 3/ Analyse des cassures de l'ADN

Les images obtenues par microscope à fluorescence après marquage par le biomarqueur γ-H2AX sont montrées sur la figure 6.

Le pourcentage de cellules positives au marquage par γ-H2AX est calculé pour chaque condition. Les résultats obtenus sont montrés sur la figure 7.

On peut y observer qu'hors traitement, une irradiation répétée aux UVA cause une augmentation de noyaux présentant le marquage par, et donc du nombre de noyaux présentant des cassures de l'ADN.

Les peaux traitées avec l'acide caféique (c), la pyridoxamine (d), et le composé comparatif Comp.1 (h) présentent un taux similaire à l'échantillon irradié (b) (ns) et nettement augmenté par rapport au contrôle non irradié (a) (***). Les peaux traitées avec les composés selon l'invention (la) (e) et (Id) (g) présentent quant à eux une augmentation du pourcentage de cellules présentant des dommages à l'ADN en comparaison du témoin, mais nettement diminuée par rapport à l'échantillon contrôle irradié (b) (**, *** respectivement). La peau traitée avec le composé (Ic) selon l'invention (f) présente avantageusement le même phénotype que l'échantillon contrôle non irradié

Les composés selon l'invention (la), (Ic) et (Id) protègent ainsi efficacement la peau contre les UVA et permettent par là-même de ralentir l'apparition des symptômes du vieillissement de la peau.

L'acide caféique, la pyridoxamine et le composé Comp.1 ne présentent quant à eux aucun effet de protection de la peau contre les UVA.

Il ressort clairement des exemples ci-avant que les composés conformes à l'invention, et tout particulièrement le composé de formule (la), et le composé de formule (Ic), combinent des propriétés leur permettant, appliqués par voie topique sur la surface cutanée, de lutter efficacement contre les agressions externes, notamment le rayonnement UV et la pollution, par des actions à la fois préventives et curatives. Ces composés agissent notamment au niveau des défenses antioxydantes et anti-inflammatoires de la peau, ils inhibent les métalloprotéases matricielles et favorisent la réparation de l'ADN. Le procédé de traitement cosmétique de la peau qui les met en œuvre s'avère de ce fait particulièrement efficace pour la prévention et la réparation des signes du vieillissement cutané.

### EXEMPLES 18 à 24 - Compositions cosmétiques

Des compositions cosmétiques conformes à l'invention présentent les compositions suivantes.

Dans ces exemples, les quantités de chaque ingrédient sont exprimées en pourcentages en poids, par rapport au poids total de la composition.

### Exemple 18 - Composition cosmétique pour un soin apaisant détoxifiant contour des yeux

Cette composition cosmétique sera utilisée 2 fois par jour matin et soir en application circulaire autour des yeux.

**Tableau 15 - Composition de soin apaisant détoxifiant contour des yeux**

| Ingrédient | % |
|---|---|
| Eau | 79,43 |
| Glycérine | 3,63 |
| Acrylates/C10-30 alkyl acrylate crosspolymer | 0,30 |
| Polyacrylate crosspolymer-6 | 0,30 |
| Sodium polyacrylate | 0,40 |
| Coco-caprylate/caprate | 3,00 |
| Isopropyl palmitate | 1,80 |
| Tridécyl trimellitate | 3,60 |
| Chlorphénesine | 0,30 |
| Alcool benzylique (et) éthylhexylglycérine (et) tocopherol | 0,90 |
| Composé (la) | 0,50 |
| Jus de feuille d'Aloe barbadensis | 5,50 |
| Nitrure de bore | 0,30 |
| Hydroxyde de sodium | 0,04 |
| | 100,000 |

### Exemple 19 - Composition cosmétique pour un lait anti-âge visage

Cette composition cosmétique sera utilisée 2 à 3 fois par jour matin et soir en application sur tout le visage hors muqueuses.

**Tableau 16 - Lait anti-âge visage**

| Ingrédient | % |
|---|---|
| Eau | 75,27 |
| Glycérine | 3,30 |
| Gomme de xanthane | 0,50 |
| Polysorbate 60 | 2,50 |
| Stéarate de sorbitan | 2,50 |
| Isononyl isononanoate | 1,50 |
| Ppg-3 benzyl éther éthylhexanoate | 2,00 |
| Phényl triméthicone | 3,00 |
| Ethylhexyl cocoate | 2,00 |
| Beurre de butyrospermum parkii | 1,00 |
| Hydroxyéthyl acrylate/sodium acryloyldiméthyl taurate copolymère & isohexadecane & polysorbate 60 | 2,00 |
| Aluminium amidon octénylsuccinate | 1,00 |
| Composé (la) | 0,10 |
| Acide hyaluronique | 2,00 |
| Parfum | 0,20 |
| Acide citrique | 0,03 |
| Chlorphénesine | 0,30 |
| Phenoxyéthanol | 0,30 |
| Ethylhexyl glycérine | 0,50 |
| | 100,00 |

### Exemple 20 - Composition cosmétique pour un soin éclaircissant détoxifiant visage

Cette composition cosmétique sera utilisée une fois par jour matin en application sur tout le visage hors muqueuses et le cou.

**Tableau 17 - Composition de soin éclaircissant détoxifiant visage**

| Ingrédient | % |
|---|---|
| Eau | 79,36 |
| Glycérine | 3,00 |
| Gomme xanthane | 0,20 |
| Polyacrylate de sodium | 0,25 |
| Alcool cétylique (et) stéarate de glycéryle (et) peg-75 stéarate (et) ceteth-20 (et) steareth-20 | 2,00 |
| Coco-caprylate/caprate | 5,00 |
| Huile de graine d'Helianthus annuus | 3,00 |
| C12-15 benzoate d'alkyle | 1,00 |
| Polyacrylamide (et) c13-14 isoparaffine (et) laureth-7 | 3,00 |
| Acrylate de sodium / acryloyldiméthyl taurate de sodium copolymere/isohexadécane/polysorbate 80 | 0,74 |
| Acide 3-o-éthyl ascorbique | 1,00 |
| Composé (la) | 0,20 |
| Parfum | 0,15 |
| Chlorphénesine | 0,30 |
| Phénoxyéthanol | 0,30 |
| Ethylhexyl glycérine | 0,50 |
| | 100,00 |

### Exemple 21 - Composition cosmétique pour une crème solaire SPF 25 - Visage et cou

Cette composition cosmétique sera utilisée avant et pendant l'exposition au soleil.

**Tableau 18 - Crème solaire SPF 25 - Visage et cou**

| Ingrédient | % |
|---|---|
| Eau | 60,65 |
| Coco-caprylate/caprate | 4,00 |
| Butyl méthoxydibenzoylméthane | 3,00 |
| Octocrylène | 10,00 |
| Salicylate d'homomenthyl | 5,00 |
| Benzophenone-3 | 3,00 |
| Diméthicone | 2,00 |
| Dioxide de titane (et) silice | 2,00 |
| Alcool cetéarylique (et) cetéaryl glucoside | 3,00 |
| Alcool arachidylique (et) alcohol béhenylique (et) arachidyl glucoside | 2,00 |
| Gomme de xanthane | 1,00 |
| Acétate de tocophéryle | 0,50 |
| Poudre Edeta B | 0,05 |
| Chlorphénésine | 0,10 |
| Glycérine | 2,00 |
| Ethylhexyl glycérine | 0,20 |
| Phénoxyéthanol | 0,30 |
| Parfum | 0,20 |
| Composé (la) | 2,00 |
| | 100,00 |

### Exemple 22 - Composition cosmétique pour un soin visage anti-âge hydratant détoxifiant

Cette composition cosmétique sera utilisée le soir avant le sommeil.

**Tableau 19 - Composition de soin visage anti-âge hydratant détoxifiant**

| Ingrédient | % |
|---|---|
| Eau | 67,55 |
| Glycérine | 3,00 |
| Chlorphénesine | 0,30 |
| Gomme xanthane | 0,50 |
| Acrylates/c10-30 alkyl acrylate crosspolymer | 0,50 |
| Solution de soude à 1 0% | 0,60 |
| Alcool cetéarylique (et) coco glucoside | 1,50 |
| Alcool arachidylique (et) alcool béhenylique (et) arachidyl glucoside | 3,50 |
| Beurre de graine de Shorea robusta | 3,00 |
| Acide stéarique | 2,00 |
| Huile de graine de Sesamum indicum (sésame) | 1,50 |
| Huile de graine de Simmondsia chinensis | 2,50 |
| Huile de graine d'Helianthus annuus | 3,50 |
| Propanediol | 4,50 |
| Diméthicone | 1,00 |
| Acétate de tocophéryle | 0,50 |
| Phenoxyéthanol | 0,30 |
| Ethylhexylglycérine | 0,50 |
| Parfum | 0,20 |
| Composé (la) | 0,05 |
| Acide hyaluronique | 2,00 |
| Resvératrol | 1,00 |
| | 100,00 |

### Exemple 23 - Composition cosmétique pour un après-rasage détoxifiant

Cette composition cosmétique sera utilisée pour après rasage des poils du visage.

**Tableau 20 - Après-rasage détoxifiant**

| Ingrédient | % |
|---|---|
| Eau | 65,30 |
| Colorant - solution à 0,01% | 1,00 |
| Lactate de menthyl | 0,50 |
| Jus de feuille d'Aloe barbadensis | 0,50 |
| Composé (la) | 2,00 |
| Phenoxyéthanol | 0,30 |
| Chlorphénesine | 0,30 |
| Ethylhexylglycérine | 0,50 |
| Polyacrylate crosspolymer-6 | 1,60 |
| Peg-11 méthyl éther diméthicone | 6,00 |
| Diméthicone | 1,00 |
| Glycérine | 3,00 |
| Ethanol | 7,00 |
| Eau | 10,00 |
| Perfect skin | 0,30 |
| Ppg-26 buteth-26 (et) peg-40 huile de ricin hydrogénée | 0,70 |
| | 100,00 |

### Exemple 24 - Composition cosmétique pour une crème teintée unifiée détoxifiante

Cette composition cosmétique sera utilisée comme un fond de teint, préférentiellement le matin ou dans la journée.

**Tableau 21 - Crème teintée unifiée détoxifiante**

| Ingrédient | % |
|---|---|
| Eau | 60,400 |
| C14-22 alcool (et) c12-20 glucoside | 2,500 |
| Diméthicone | 0,500 |
| C12-15 alkyl benzoate | 5,000 |
| Sodium acrylate/sodium acryloyldiméthyl taurate copolymère/isohexadécane/polysorbate 80 | 3,500 |
| Diméthicone | 2,000 |
| Cyclopentasiloxane | 3,000 |
| Ci 77492 (et) glycérine (et) eau (et) gomme xanthane (et) citrate de sodium | 0,830 |
| ci 77491 (et) glycérine (et) eau (et) gomme xanthane (et) citrate de sodium | 0,860 |
| Ci 77499 (et) glycérine (et) eau (et) gomme xanthane (et) citrate de sodium | 0,190 |
| Ci 77891 (et) glycérine (et) eau (et) gomme xanthane (et) citrate de sodium | 19,200 |
| Acide 3-o-éthyl ascorbique | 1,000 |
| Composé (la) | 0,200 |
| Ethylhexyl glycérine | 0,300 |
| Phénoxyéthanol | 0,500 |
| Parfum | 0,020 |
| | 100,00 |

## Revendications

1. Procédé de traitement cosmétique non-thérapeutique de la peau d'un individu, **caractérisé en ce qu'**il comprend l'administration audit individu d'une composition contenant, dans un véhicule cosmétiquement acceptable, un composé de formule générale (I') : 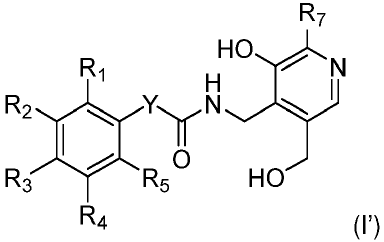 dans laquelle :
R₁, R₂, R₃, R₄, R₅, et R₇, identiques ou différents, représentent chacun un atome d'hydrogène, un atome d'halogène, un groupement hydroxyle, un radical hydrocarboné linéaire ou ramifié, saturé ou insaturé, en C1-C16, ou un radical -OR' où R' représente un radical hydrocarboné linéaire ou ramifié, saturé ou insaturé, en C1-C16,
au moins un groupement parmi R₁, R₂, R₃, R₄ et R₅ représentant un groupement hydroxyle,
et Y représente une liaison covalente ou un radical alcényle en C2-C4,
ou un de ses sels.

2. Procédé selon la revendication 1, selon lequel l'administration de ladite composition audit individu est effectuée par application de ladite composition par voie topique sur la peau dudit individu.

3. Procédé selon l'une quelconque des revendications 1 à 2, selon lequel Y représente un radical alcényle en C2.

4. Procédé selon l'une quelconque des revendications 1 à 3, selon lequel, dans la formule (I'), au moins un substituant parmi R₂, R₃ et R₄ représente un groupement hydroxyle.

5. Procédé selon l'une quelconque des revendications 1 à 2, selon lequel ledit composé de formule générale (I') répond à la formule (I") : 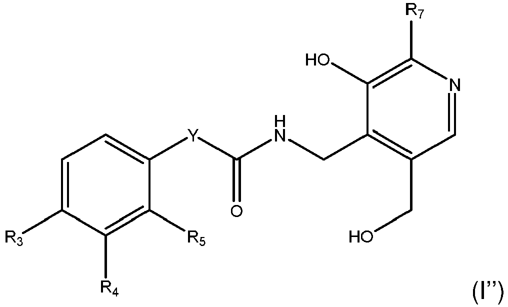 dans laquelle
R₃, R₄, R₅ représentent chacun un atome d'hydrogène, un atome d'halogène, un groupement hydroxyle, un radical hydrocarboné linéaire ou ramifié, saturé ou insaturé, en C1-C16, ou un radical -OR' où R' représente un radical hydrocarboné linéaire ou ramifié, saturé ou insaturé, en C1-C16,
au moins un premier groupement parmi R₃, R₄ et R₅ représentant un groupement hydroxyle et au moins un deuxième groupement parmi R₃, R₄ et R₅ représentant un groupement hydroxyle ou un radical -OR' où R' représente un radical hydrocarboné linéaire ou ramifié, saturé ou insaturé, en C1-C4,
R₇ représente un radical hydrocarboné linéaire ou ramifié, saturé ou insaturé, en C1-C4,
et Y représente une liaison covalente ou un radical alcényle en C2-C4.

6. Procédé selon la revendication 5, selon lequel, dans la formule (I"), au moins un substituant parmi R₃ et R₄ représente un groupement hydroxyle.

7. Procédé selon l'une quelconque des revendications 1 à 3, selon lequel ledit composé de formule générale (I') répond à la formule (I"') : 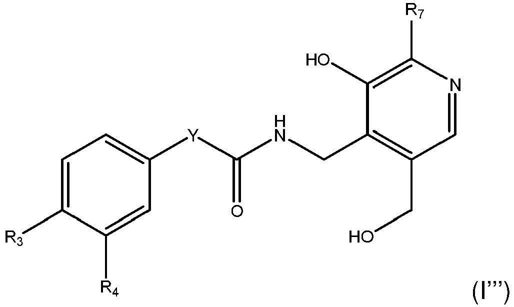 dans laquelle
R₃ représente un groupement hydroxyle, ou un radical -OR' où R' représente un radical hydrocarboné linéaire ou ramifié, saturé ou insaturé, en C1-C16,
R₄ représente un atome d'hydrogène ou un groupement hydroxyle,
et R₇ représente un radical hydrocarboné linéaire ou ramifié, saturé ou insaturé, en C1-C4.

8. Procédé selon la revendication 7, selon lequel, dans la formule (I"'), R₄ représente un groupement hydroxyle.

9. Procédé selon l'une quelconque des revendications 1 à 8, selon lequel ledit composé de formule générale (I') répond à la formule (la) :

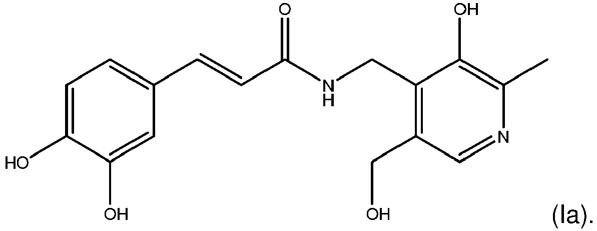

10. Procédé selon l'une quelconque des revendications 1 à 8, selon lequel ledit composé de formule générale (I') répond à la formule (Ic) :

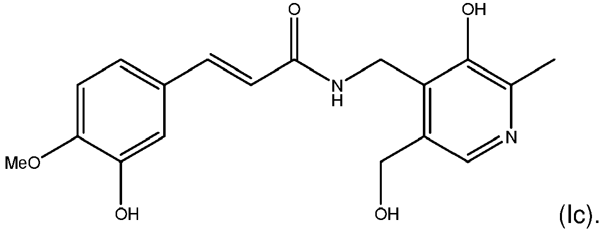

11. Utilisation d'un composé de formule générale (I') tel que défini dans l'une des revendications 1 à 10, ou d'un de ses sels, pour le traitement cosmétique non-thérapeutique de la peau d'un individu, notamment pour la prévention et/ou la réparation des signes du vieillissement cutané.

12. Utilisation selon la revendication 11, selon laquelle ledit composé est appliqué par voie topique sur la peau dudit individu.

13. Composition cosmétique, **caractérisé en ce qu'**elle contient un composé de formule générale (I') tel que défini dans l'une des revendications 1 à 10, ou un de ses sels, à l'exclusion des composés suivants :
3-(2,4-dihydroxyphényl)-N-(3-hydroxy-5-hydroxyméthyl-2-méthyl-pyridin-4-ylméthyl)-acrylamide
3-(3,5-dihydroxyphényl)-N-(3-hydroxy-5-hydroxyméthyl-2-méthyl-pyridin-4-ylméthyl)-acrylamide
3-(2,6-dihydroxyphényl)-N-(3-hydroxy-5-hydroxyméthyl-2-méthyl-pyridin-4-ylméthyl)-acrylamide,
dans un véhicule cosmétiquement acceptable.

## Patentansprüche

1. Nicht-therapeutisches kosmetisches Behandlungsverfahren der Haut eines Individuums, **dadurch gekennzeichnet, dass** es umfasst, dem Individuum eine Zusammensetzung zu verabreichen, die in einem kosmetisch akzeptablen Träger eine Verbindung enthält mit der allgemeinen Formel (I'): 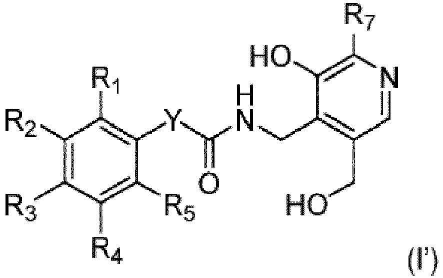 in welcher:
R₁, R₂, R₃, R₄, R₅ und R₇, identisch oder unterschiedlich, jeweils ein Wasserstoffatom, ein Halogenatom, eine Hydroxylgruppe, ein lineares oder verzweigtes, gesättigtes oder ungesättigtes C₁-C₁₆-Kohlenwasserstoffradikal oder ein -OR'-Radikal darstellen, wobei R' ein lineares oder verzweigtes, gesättigtes oder ungesättigtes C₁-C₁₆-Kohlenwasserstoffradikal darstellt,
mindestens eine Gruppe von R₁, R₂, R₃, R₄ und R₅ eine Hydroxylgruppe darstellt,
und Y eine kovalente Bindung oder ein C₂-C₄-Alkenylradikal darstellt,
oder eines ihrer Salze.

2. Verfahren nach Anspruch 1, wobei die Zusammensetzung dem Individuum durch Auftragen der Zusammensetzung auf topischem Weg auf die Haut des Individuums verabreicht wird.

3. Verfahren nach einem der Ansprüche 1 bis 2, wobei Y ein C₂-Alkenylradikal darstellt.

4. Verfahren nach einem der Ansprüche 1 bis 3, wobei in der Formel (I') mindestens ein Substituent von R₂, R₃ und R₄ eine Hydroxylgruppe darstellt.

5. Verfahren nach einem der Ansprüche 1 bis 2, wobei die Verbindung der allgemeinen Formel (I') der Formel (I") entspricht: 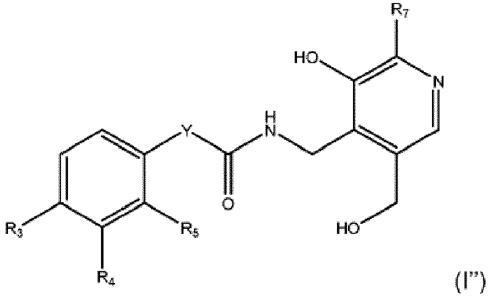 in welcher:
R₃, R₄, R₅ jeweils ein Wasserstoffatom, ein Halogenatom, eine Hydroxylgruppe, ein lineares oder verzweigtes, gesättigtes oder ungesättigtes C₁-C₁₆-Kohlenwasserstoffradikal oder ein -OR'-Radikal darstellen, wobei R' ein lineares oder verzweigtes, gesättigtes oder ungesättigtes C₁-C₁₆-Kohlenwasserstoffradikal darstellt,
mindestens eine erste Gruppe von R₃, R₄ und R₅ eine Hydroxylgruppe darstellt, und mindestens eine zweite Gruppe von R₃, R₄ und R₅ eine Hydroxylgruppe oder ein - OR'-Radikal darstellt, wobei R' ein lineares oder verzweigtes, gesättigtes oder ungesättigtes C₁-C₄-Kohlenwasserstoffradikal darstellt,
R₇ ein lineares oder verzweigtes, gesättigtes oder ungesättigtes C₁-C₄-Kohlenwasserstoffradikal darstellt,
und Y eine kovalente Bindung oder ein C₂-C₄-Alkenylradikal darstellt.

6. Verfahren nach Anspruch 5, wobei in der Formel (I") mindestens ein Substituent von R₃ und R₄ eine Hydroxylgruppe darstellt.

7. Verfahren nach einem der Ansprüche 1 bis 3, wobei die Verbindung der allgemeinen Formel (I') der Formel (I"') entspricht: 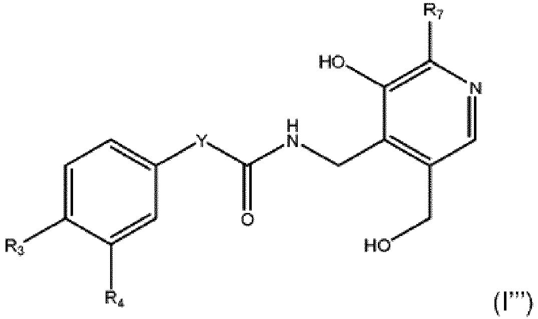 in welcher:
R₃ eine Hydroxylgruppe oder ein -OR'-Radikal darstellt, wobei R' ein lineares oder verzweigtes, gesättigtes oder ungesättigtes C₁-C₁₆-Kohlenwasserstoffradikal darstellt,
R₄ ein Wasserstoffatom oder eine Hydroxylgruppe darstellt,
und R₇ ein lineares oder verzweigtes, gesättigtes oder ungesättigtes C₁-C₄-Kohlenwasserstoffradikal darstellt.

8. Verfahren nach Anspruch 7, wobei in der Formel (I"') R₄ eine Hydroxylgruppe darstellt.

9. Verfahren nach einem der Ansprüche 1 bis 8, wobei die Verbindung der allgemeinen Formel (I') der Formel (Ia) entspricht:

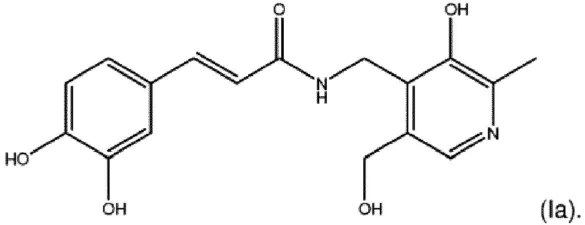

10. Verfahren nach einem der Ansprüche 1 bis 8, wobei die Verbindung der allgemeinen Formel (I') der Formel (Ic) entspricht:

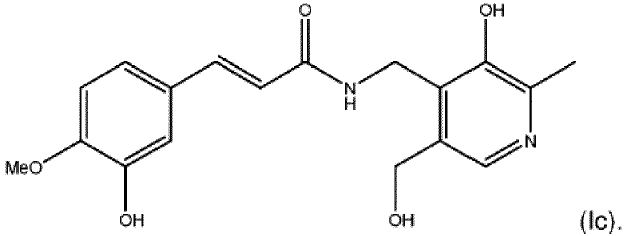

11. Verwendung einer Verbindung der allgemeinen Formel (I') nach einem der Ansprüche 1 bis 10 oder eines ihrer Salze zur nicht-therapeutischen kosmetischen Behandlung der Haut eines Individuums, insbesondere zur Vorbeugung und/oder Reparatur der Zeichen der Hautalterung.

12. Verwendung nach Anspruch 11, wobei die Verbindung auf topischem Weg auf die Haut des Individuums aufgetragen wird.

13. Kosmetische Zusammensetzung, **dadurch gekennzeichnet, dass** sie eine Verbindung der allgemeinen Formel (I') nach einen der Ansprüche 1 bis 10 oder eines ihrer Salze enthält, mit Ausnahme der folgenden Verbindungen:
3-(2,4-Dihydroxyphenyl)-N-(3-hydroxy-5-hydroxymethyl-2-methyl-pyridin-4-ylmethyl)-acrylamid
3-(3,5-Dihydroxyphenyl)-N-(3-hydroxy-5-hydroxymethyl-2-methyl-pyridin-4-ylmethyl)-acrylamid
3-(2,6-Dihydroxyphenyl)-N-(3-hydroxy-5-hydroxymethyl-2-methyl-pyridin-4-ylmethyl)-acrylamid,
in einem kosmetisch akzeptablen Träger.

## Claims

1. Method for non-therapeutic cosmetic treatment of the skin of an individual, **characterised in that** it comprises the administration, to said individual, of a composition containing, in a cosmetically acceptable vehicle, a compound having the general formula (I'): 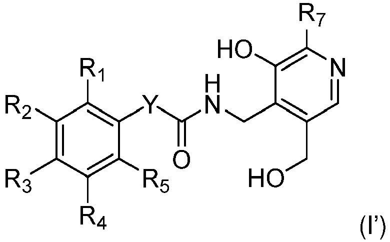 wherein:
R₁, R₂, R₃, R₄, R₅, and R₇, identical or different, each represent a hydrogen atom, a halogen atom, a hydroxyl group, a linear or branched, saturated or unsaturated, C1-C16 hydrocarbon radical, or an -OR' radical in which R' represents a linear or branched, saturated or unsaturated, C1-C16 hydrocarbon radical,
at least one group out of R₁, R₂, R₃, R₄ and R₅ representing a hydroxyl group,
and Y represents a covalent bond or a C2-C4 alkenyl radical,
or one of the salts thereof.

2. Method according to claim 1, wherein the administration of said composition to said individual is carried out by applying said composition topically onto the skin of said individual.

3. Method according to any one of claims 1 to 2, wherein Y represents a C2 alkenyl radical.

4. Method according to any one of claims 1 to 3, wherein, in formula (I'), at least one substituent out of R₂, R₃ and R₄ represents a hydroxyl group.

5. Method according to any one of claims 1 to 2, wherein said compound of general formula (I') has the formula (I"): 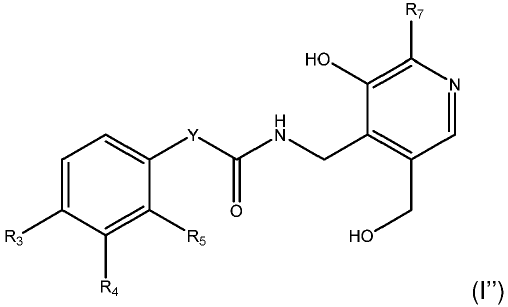 wherein
R₃, R₄, R₅ each represent a hydrogen atom, a halogen atom, a hydroxyl group, a linear or branched, saturated or unsaturated, C1-C16 hydrocarbon radical, or an - OR' radical in which R' represents a linear or branched, saturated or unsaturated, C1-C16 hydrocarbon radical,
at least one first group out of R₃, R₄ and R₅ representing a hydroxyl group and at least one second group out of R₃, R₄ and R₅ representing a hydroxyl group or an -OR' radical in which R' represents a linear or branched, saturated or unsaturated, C1 - C4 hydrocarbon radical,
R₇ represents a linear or branched, saturated or unsaturated, C1-C4 hydrocarbon radical,
and Y represents a covalent bond or a C2-C4 alkenyl radical.

6. Method according to claim 5, wherein, in the formula (I"), at least one substituent out of R₃ and R₄ represents a hydroxyl group.

7. Method according to any one of claims 1 to 3, wherein said compound of general formula (I') has the formula (I"'): 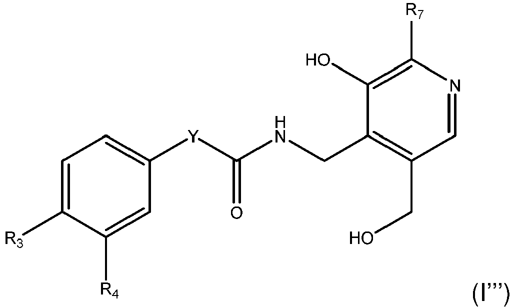 wherein
R₃ represents a hydroxyl group, or an -OR' radical in which R' represents a linear or branched, saturated or unsaturated, C1-C16 hydrocarbon radical,
R₄ represents a hydrogen atom or a hydroxyl group,
and R₇ represents a linear or branched, saturated or unsaturated, C1-C4 hydrocarbon radical.

8. Method according to claim 7, wherein, in the formula (I"'), R₄ represents a hydroxyl group.

9. Method according to any one of claims 1 to 8, wherein said compound of general formula (I') has the formula (Ia):

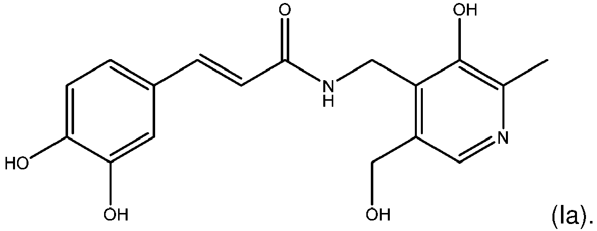

10. Method according to any one of claims 1 to 8, wherein said compound of general formula (I') has the formula (Ic):

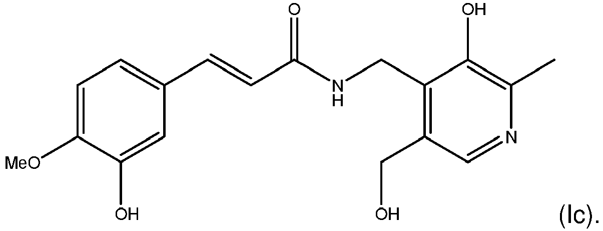

11. Use of a compound having the general formula (I') as defined in one of claims 1 to 10, or of one of the salts thereof, for the non-therapeutic cosmetic treatment of the skin of an individual, in particular for the prevention and/or the repair of the signs of ageing of the skin.

12. Use according to claim 11, wherein said compound is applied topically onto the skin of said individual.

13. Cosmetic composition, **characterised in that** it contains a compound having the general formula (I') as defined in one of claims 1 to 10, or one of the salts thereof, with the exclusion of the following compounds:
3-(2,4-dihydroxyphenyl)-N-(3-hydroxy-5-hydroxymethyl-2-methyl-pyridin-4-ylmethyl)-acrylamide
3-(3,5-dihydroxyphenyl)-N-(3-hydroxy-5-hydroxymethyl-2-methyl-pyridin-4-ylmethyl)-acrylamide
3-(2,6-dihydroxyphenyl)-N-(3-hydroxy-5-hydroxymethyl-2-methyl-pyridin-4-ylmethyl)-acrylamide,
in a cosmetically acceptable vehicle.
